# EUROPEAN PATENT APPLICATION

(11) **EP 4 726 392 A1**
(43) Date of publication of application: **15.04.2026**
(21) Application number: 24205670.3
(22) Date of filing: 09.10.2024
(51) Int. Cl.: G01N 33/68

(54) **BLOOD TEST FOR PREDICTING SPORTS-ASSOCIATED INJURIES**

(71) Applicant: Knappschaft Kliniken Universitätsklinikum Bochum GmbH, 44892 Bochum (DE)
(72) Inventor: Adamzik, Michael, 44892 Bochum (DE); Ziehe, Dominik, 44892 Bochum (DE); Koos, Björn, 44892 Bochum (DE); Krüger, Karsten, 35390 Giessen (DE)
(74) Representative: Vossius & Partner Patentanwälte Rechtsanwälte mbB

(57) **Abstract**

The present invention concerns predictive methods and kits for non-medical uses, for example in an athletic training, as well as for medical uses, e.g., in the rehabilitation of a patient. In particular, the present invention concerns methods of predicting sports-associated injuries such as muscle, tendon and/or ligament injuries. Therefore, the present invention relates to a method of predicting a muscle, tendon and/or ligament injury in a subject, said method comprising measuring the level of at least one biomarker for mitochondrial resilience and/or functionality in a blood sample from the subject, (i) wherein (a) level(s) of said biomarker(s) below (a) respective threshold(s) is/are indicative of a high likelihood that a muscle, tendon and/or ligament injury will occur in the subject, and/or (ii) wherein (a) level(s) of said biomarker(s) equal to or above (a) respective threshold(s) is/are indicative of a low likelihood that a muscle, tendon and/or ligament injury will occur in the subject. Furthermore, the present invention relates to a method of detecting an abnormal stress level of a biomarker for mitochondrial resilience and/or functionality in a blood sample from a subject. The present invention also relates to methods for determining the performance and/or strength level of a subject, or for predicting the development of the performance and/or strength of a subject. Additionally, the present invention relates to a kit comprising at least one means for measuring the level(s) of at least one biomarker for mitochondrial resilience and/or functionality, preferably for use in the methods mentioned herein above. Furthermore, these methods may be used for preventing a muscle, tendon and/or ligament injury in a subject, for optimizing the performance and/or training of an athlete, or for optimizing the rehabilitation of a patient.

## Description

The present invention concerns predictive methods and kits for non-medical uses, for example in an athletic training, as well as for medical uses, e.g., in the rehabilitation of a patient. In particular, the present invention concerns methods of predicting sports-associated injuries such as muscle, tendon and/or ligament injuries. Therefore, the present invention relates to a method of predicting a muscle, tendon and/or ligament injury in a subject, said method comprising measuring the level of at least one biomarker for mitochondrial resilience and/or functionality in a blood sample from the subject, (i) wherein (a) level(s) of said biomarker(s) below (a) respective threshold(s) is/are indicative of a high likelihood that a muscle, tendon and/or ligament injury will occur in the subject, and/or (ii) wherein (a) level(s) of said biomarker(s) equal to or above (a) respective threshold(s) is/are indicative of a low likelihood that a muscle, tendon and/or ligament injury will occur in the subject. Furthermore, the present invention relates to a method of detecting an abnormal stress level of a biomarker for mitochondrial resilience and/or functionality in a blood sample from a subject. The present invention also relates to methods for determining the performance and/or strength level of a subject, or for predicting the development of the performance and/or strength of a subject. Additionally, the present invention relates to a kit comprising at least one means for measuring the level(s) of at least one biomarker for mitochondrial resilience and/or functionality, preferably for use in the methods mentioned herein above. Furthermore, these methods may be used for preventing a muscle, tendon and/or ligament injury in a subject, for optimizing the performance and/or training of an athlete, or for optimizing the rehabilitation of a patient.

In any kinds of sports, including amateur sports, professional sports and elite sports, it is desirable to achieve and/or maintain the level of performance aimed for. Especially in professional and elite sports, it is of great importance that the individual athletes achieve and maintain a stable level of performance over a longer period of time. For example, ambitious sporting goals can only be achieved if the athletes can repeatedly push themselves to their individual performance limits over the course of a season. Optimal care and the best possible training are important for exploiting performance reserves. Especially in high performance classes, where players are particularly motivated and willing to perform, there is an increased risk of injury with potentially long downtimes. Also individuals only occasionally performing physical exercises or sport in form of amateur sports may suffer from injuries. It is therefore desirable that an increased propensity for an injury can be identified, and protective measures can be taken to prevent an injury from developing in the first place. Once an injury has occurred, it is very important for an athlete (and his/her coach) to know the best possible time when the athlete can return to training or to participate (again) in a competition and at what intensity. It is thus important to find the right balance between recovery and training phases for athletes to minimize the risk of (re)-injury.

Blood-based, exercise-sensitive biomarkers from the field of chemokines or cytokines have become established in sports in recent decades, which provide information about an athlete's exertion/stress level. These biomarkers are supported by accompanying subjective tests to determine cognitive performance (e.g., Stroop color and word test) or the subjective perception of pain (McGill pain scale). In addition, the analysis of maximum oxygen uptake (VO₂max) is used to determine the cardiorespiratory fitness of an athlete. This indicates the maximum amount of oxygen that can be absorbed by the body during an intensive training session. However, VO₂max is subject to strong individual differences, such as body weight. The aforementioned biomarkers can only reflect an athlete's level of exertion/stress and training situation to a limited extent.

It is particularly of high relevance in sports (but also in medical situations such a rehabilitation) to predict and prevent muscle injuries that can occur due to overstraining.

To date, there are attempts to predict muscle injuries through a series of markers; Damas (2016), Int J Sports Med 2016; 37(08). In particular, at least one of the following processes is usually examined to predict muscle injuries: a) Loss of myofibril integrity; b) Extracellular matrix remodeling or failure of excitation-contraction coupling (reflected in reduced muscle strength); c) Connective tissue damage and decreases in range of motion; d) Membrane damage (leading to the leakage of muscle proteins into the bloodstream such as creatine kinase (CK)); e) Muscle swelling (increase in limb circumference); f) Inflammatory events (promoting the accumulation of leukocytes in the muscle), as determined, in particular, by measuring cytokine/chemokine levels.

However, the current approaches for predicting muscle injuries are cumbersome and/or not reliable. In particular, the biomarkers established to date for predicting muscle injuries are subject to strong inter-individual fluctuations. For example, some athletes show a strong physical response (potentially leading to a muscle injury) to even the smallest changes in cytokine concentration, while others show hardly any physical response despite basal high cytokine levels.

Hence, there is still a need for means and methods for reliably determining a propensity for sport-associated injuries such as muscle, tendon and/or ligament injuries.

The invention relates to the embodiments as characterized in the claims and as described herein below.

Accordingly, the present invention relates to a method of predicting a muscle, tendon and/or ligament injury in a subject, said method comprising measuring the level of at least one biomarker for mitochondrial resilience and/or functionality in a blood sample from the subject,
(i) wherein (a) level(s) of said biomarker(s) below (a) respective threshold(s) is/are indicative of a high likelihood that a muscle, tendon and/or ligament injury will occur in the subject, and/or
(ii) wherein (a) level(s) of said biomarker(s) equal to or above (a) respective threshold(s) is/are indicative of a low likelihood that a muscle, tendon and/or ligament injury will occur in the subject.

The present invention is, *inter alia,* based on the surprising finding that biomarkers for mitochondrial resilience and/or functionality, in particular ATP and/or the TFAM-TFB2M complex, measured in blood can be used for predicting sports-associated injuries such as injuries of muscles, tendons and/or ligaments. Specifically, as illustrated in the appended non-limiting Examples, it has been surprisingly found, that the level of ATP in leukocytes predicts the occurrence of such a sports-associated injury in professional football players within 10 days with a very high accuracy (AUC of 83-95%), when used in combination with the level of the TFAM-TFB2M complex (i.e. the number of interactions of TFAM protein with TFB2M protein) in leukocytes and/or the level of at least one cytokine such as IL-6, IL-8, IL-10 and/or TNF-o in blood. This constitutes a significant improvement over purely cytokine-based blood tests which achieved at most an AUC of up to 80% in predicting such sports-associated injuries and could not be further improved by measuring the level of more than two cytokines; see Example 1.

As used herein, the term "mitochondrial functionality" refers to the ability of mitochondria to generate energy in form of ATP (adenosine triphosphate).

The term "mitochondrial resilience", as used herein, refers to the ability of mitochondria to re-establish or increase the generation of ATP after a stop or decrease of the generation of ATP due to a stress perturbation, respectively. Stress perturbations which lead to a stop or decrease of the generation of ATP by mitochondria include, *inter alia,* infections (e.g., viral or bacterial infections) and excessive athletic training.

TFAM (mitochondrial transcription factor A) is a major promoter of mitochondrial biogenesis that plays a central role in the mitochondrial core transcription initiation complex in combination with TFB2M (mitochondrial transcription factor B2); Ramachandran (2017), Nucleic Acids Res 45. Thus, TFAM, specifically when comprised in the TFAM-TFB2M complex, is responsible for mitochondrial repair, proliferation and expression. The TFAM-TFB2M complex has been further established as a biomarker of mitochondrial functionality, regenerative ability, and vitality; see WO 2022/106666 and Rahmel (2020), Sci Rep 3;10(1). Therefore, TFAM (esp. in complex with TFB2M) can be used as a biomarker for mitochondrial resilience and/or functionality.

ATP is the functional output of mitochondria that provides energy to drive and support many processes in living cells. Accordingly, ATP can be also used as a biomarker for mitochondrial resilience and/or functionality.

Although other samples, in particular tissue samples (e.g., from muscles) may be also used instead of or in addition to blood samples in the present invention, the use of blood samples is particularly versatile and minimally invasive and thus particularly advantageous. Hence, blood samples are particularly preferred samples in context of the present invention. Most preferably, the level(s) of the biomarker(s) for mitochondrial resilience and/or functionality, e.g., ATP and/or the interaction of TFAM with TFB2M, is/are measured in at least one leukocyte such as a peripheral blood mononuclear cell (PBMC), of the blood sample.

Therefore, the present invention provides, *inter alia,* a predictive blood test that allows to accurately determine the propensity for and thus to predict sports-associated injuries such as muscle, tendon and/or ligament injuries, and that can be easily and routinely applied in various non-medical or medical situations.

For example, the predictive method of the invention can be used for optimizing the training and/or performance of an athlete such as, *inter alia,* a football (soccer) player. In particular, the method allows to identify an increased or high propensity/likelihood/risk of an athlete to get injured at a muscle, tendon and/or ligament, and to advise the athlete on his/her next training/physical exercises. Specifically, in case of a high likelihood that a muscle, tendon and/or ligament injury will occur in the athlete, the athlete can be advised/instructed to perform no or only an easy physical exercise or athletic training, and/or to discontinue or reduce a physical exercise/athletic training, in particular, a strenuous physical exercise/athletic training. Like this, a muscle, tendon and/or ligament injury may be prevented in the athlete.

In case of a low likelihood that a muscle, tendon and/or ligament injury will occur in the athlete, the athlete can be advised/instructed to perform a physical exercise or athletic training, preferably a strenuous physical exercise/athletic training, and/or to continue or extend a physical exercise/athletic training, preferably a strenuous physical exercise/athletic training.

Therefore, the present invention allows a subject such as an athlete to find the right balance between recovery and training/exercising phases and to reduce the risk of a (further) injury, e.g., a muscle, tendon and/or ligament injury. Especially for professional or elite athletes this is very important in order to achieve and maintain a stable level of performance, in particular over an entire season. However, the present invention can be useful for anyone who, at least occasionally, performs a physical exercise or is interested in doing so, or in amateur sports.

Moreover, the predictive method of the invention can be also used for optimizing the rehabilitation of a patient that is undergoing or scheduled for rehabilitation, e.g., due to a stroke, a heart attack, sepsis, long COVID, or a previous muscle, tendon and/or ligament injury.

In case of a high likelihood that a muscle, tendon and/or ligament injury will occur in the patient, the patient can be advised/instructed to perform no or only an easy physical (rehabilitation) exercise, and/or to discontinue or reduce a physical (rehabilitation) exercise, in particular a strenuous physical (rehabilitation) exercise. Like this, a (further) muscle, tendon and/or ligament injury may be prevented in the patient.

In case of a low likelihood that a muscle, tendon and/or ligament injury will occur in the patient, further the patient can be advised/instructed to perform a physical (rehabilitation) exercise, preferably a strenuous physical (rehabilitation) exercise, and/or to continue or extend a physical (rehabilitation) exercise, preferably a strenuous physical (rehabilitation) exercise. Therefore, the present invention allows a rehabilitation patient to find the right balance between recovery and exercising phases and to reduce the risk of a (further) injury, e.g., a muscle, tendon and/or ligament injury. This may increase the efficacy/efficiency of the rehabilitation program of the patient and thereby lead to a faster recovery/convalescence of the patient and/or a higher quality of life from which the patient and his/her surroundings as well as the health system can benefit.

As used herein, pairs of terms such as "low" and "high", or "low" and "strong", are understood for the skilled artisan as being relative to each other. For example, a condition (e.g., a certain level of a biomarker) that is indicative of a high likelihood that a muscle, tendon and/or ligament injury will occur in a subject means that under this condition the likelihood is higher that such an injury will occur as compared to a condition that is indicative of a low likelihood that such an injury will occur. Accordingly, in this example, a condition (e.g., a certain level of a biomarker) that is indicative of a low likelihood that a muscle, tendon and/or ligament injury will occur in a subject means that under this condition the likelihood is lower that such an injury will occur as compared to a condition that is indicative of a high likelihood that such an injury will occur. The person skilled in the art has no difficulties in making proper comparisons. In particular, the person skilled in the art understands that conditions should be comparable to each other. For example, the period in which the occurrence of an injury is predicted should be constant across conditions.

The biomarker(s) for mitochondrial resilience and/or functionality according to the invention may be associated positively with mitochondrial repair, mitochondrial biogenesis, mitochondrial transcription and/or cellular respiration.

In some preferred embodiments of the present invention, the biomarker(s) for mitochondrial resilience and/or functionality comprise(s) adenosine triphosphate (ATP). In particular, herein and in context of the invention, the level of ATP may correspond to the level of ATP in leukocytes such as PBMCs.

The level of ATP (e.g. in leukocytes or PBMCs) can be measured by routine methods known in the art. For example, the level of ATP can be measured by an assay wherein luciferin, in particular D-luciferin, is converted to oxyluciferin in the presence of oxygen and ATP. Furthermore, the level of ATP can be measured by using an immunoassay employing an antibody directed against ATP (e.g., anti-ATP, clone AA 44-220 from antibodies-online.com) such as ELISA or flow cytometry. It is also possible to measure the level of ATP label-free in real-time, e.g., by using the Seahorse system from Agilent. Preferably, the level of ATP is measured by an assay wherein luciferin is converted to oxyluciferin in the presence of oxygen and ATP, preferably as illustrated in the appended non-limiting Examples.

In further preferred embodiments of the present invention, the biomarker(s) for mitochondrial resilience and/or functionality comprise(s) mitochondrial transcription factor A (TFAM).

The term "mitochondrial transcription factor A" or "TFAM", as used herein and in context of the present invention, refers, preferably, to active and/or mature TFAM protein. Thus, preferably herein and in context of the present invention, the level of TFAM is the level of active and/or mature TFAM protein which may, preferably, correspond to the number of interactions of TFAM protein with TFB2M protein (i.e. the amount of TFAM-TFB2M complex), the amount of mature TFAM protein, and/or the ratio of the amount of mature TFAM protein over the amount of immature TFAM protein, in particular, in leukocytes such as PBMCs. More preferably herein, the level of TFAM corresponds to the number of interactions of TFAM protein with TFB2M protein (i.e., the amount of TFAM-TFB2M complex). The level of active and/or mature TFAM protein can be determined as described herein and as illustrated in the non-limiting Examples, or as described in WO 2022/106666 which is incorporated herein by reference in its entirety.

It is thought that, after cytosolic synthesis as an immature precursor protein (~29kDa), TFAM is shuttled to the mitochondria, crossing the outer and inner membranes. The mature TFAM (~24kDa) is then generated by cleavage of a targeting sequence (~5kDa) by a processing peptidase in the mitochondrial matrix (Garstka (2003), Nucleic Acids Res 31; Prasai (2017), Pathophysiology 24).

Thus, as used herein, the mature and/or active TFAM protein has, preferably, a molecular mass of about 22 to 26 kDa, more preferably about 24 kDa. Furthermore, as used herein, the immature TFAM protein has, preferably, a molecular mass of about 27 to 30 kDa, more preferably about 29 kDa. The immature TFAM protein may be also considered as "inactive", for example, because it does not bind TFB2M protein within the mitochondrial core transcription initiation complex. The immature TFAM protein may be also considered as "inactive" because it is not at the location where TFAM is active (i.e. in the mitochondrial matrix and/or at the mitochondrial genome).

As used herein and in the context of the present invention, the mature and/or active TFAM protein may refer to:
(i) a human mature TFAM protein with a sequence set forth in SEQ ID NO:4 or SEQ ID NO:8, preferably SEQ ID NO:4;
(ii) a protein that is encoded by a nucleotide sequence set forth in SEQ ID NO:3 or SEQ ID NO:7, preferably SEQ ID NO:3;
(iii) a mature TFAM protein that is orthologous to the human mature TFAM protein of (i);
(iv) a protein that is encoded by a nucleotide sequence which is orthologous to the nucleotide sequence described in (ii); and/or
(v) a protein that has at least 60%, 70%, 80% or 90%, preferably at least 90%, 95%, 98% or 99%, e.g. 95 %, 96 %, 97 %, 98 %, or 99 % sequence identity to the protein of any one of (i) to (iv), preferably (i) or (ii), more preferably (i).

In particular, the mature and/or active TFAM protein may be a human mature TFAM protein with a sequence set forth in SEQ ID NO:4 or SEQ ID NO:8, preferably SEQ ID NO:4, and/or a protein that has at least 60%, 70%, 80% or 90%, preferably at least 90%, 95%, 98% or 99%, e.g. 95 %, 96 %, 97 %, 98 %, or 99 % sequence identity to said human mature TFAM protein (preferably to the sequence set forth in SEQ ID NO:4). More preferably, the mature and/or active TFAM protein has a sequence set forth in SEQ ID NO:4 or SEQ ID NO:8, preferably SEQ ID NO:4.

As used herein and in the context of the present invention, the immature TFAM protein, may refer to:
(i) a human immature TFAM protein with a sequence set forth in SEQ ID NO:2 or SEQ ID NO:6, preferably SEQ ID NO:2;
(ii) a protein that is encoded by a nucleotide sequence set forth in SEQ ID NO:1 or SEQ ID NO:5, preferably SEQ ID NO:1;
(iii) a mature TFAM protein that is orthologous to the human immature TFAM protein of (i);
(iv) a protein that is encoded by a nucleotide sequence which is orthologous to the nucleotide sequence described in (ii); and/or
(v) a protein that has at least 60%, 70%, 80% or 90%, preferably at least 90%, 95%, 98% or 99%, e.g. 95 %, 96 %, 97 %, 98 %, or 99 % sequence identity to the protein of any one of (i) to (iv), preferably (i) or (ii), more preferably (i).

In particular, the immature TFAM protein may be a human immature TFAM protein with a sequence set forth in SEQ ID NO:2 or SEQ ID NO:6, preferably SEQ ID NO:2, and/or a protein that has at least 60%, 70%, 80% or 90%, preferably at least 90%, 95%, 98% or 99%, e.g. 95 %, 96 %, 97 %, 98 %, or 99 % sequence identity to said human immature TFAM protein (preferably to the sequence set forth in SEQ ID NO:2). More preferably, the immature TFAM protein has a sequence set forth in SEQ ID NO:2 or SEQ ID NO:6, preferably SEQ ID NO:2.

In particular, the immature TFAM protein, as used herein, contains a N-terminal part that is cleaved off when the mature TFAM protein is generated. Accordingly, the mature TFAM protein, as used herein, does, in particular, not contain, i.e. not at the N-terminal end, a sequence corresponding to the N-terminal part of the immature TFAM protein that is cleaved off when the mature TFAM protein is generated. For example, said N-terminal part of the immature TFAM protein may have the amino acid sequence set forth in positions 1 to 42 of SEQ ID NO:2 (i.e. as set forth in SEQ ID NO:22), an amino acid sequence encoded by the nucleic acid sequence set forth in positions 1 to 126 of SEQ ID NO:1 (i.e. as set forth in SEQ ID NO:21), or an amino acid sequence that has at least 60%, 70%, 80% or 90%, preferably at least 90%, 95%, 98% or 99%, e.g. 95 %, 96 %, 97 %, 98 %, or 99 % sequence identity to any of said amino acid sequences (preferably to the sequence set forth in SEQ ID NO:22).

Herein and in context of the present invention, the term "mitochondrial transcription factor B2" or "TFB2M" refers, preferably, to mature and/or active TFB2M protein.

As used herein and in the context of the present invention, the mature and/or active TFB2M protein, may refer to:
(i) a human mature TFB2M protein with a sequence set forth in SEQ ID NO:12 or SEQ ID NO:16, preferably SEQ ID NO:12;
(ii) a protein that is encoded by a nucleotide sequence set forth in SEQ ID NO:11 or SEQ ID NO:15, preferably SEQ ID NO:11;
(iii) a mature TFB2M protein that is orthologous to the human mature TFB2M protein of (i);
(iv) a protein that is encoded by a nucleotide sequence which is orthologous to the nucleotide sequence described in (ii); and/or
(v) a protein that has at least 60%, 70%, 80% or 90%, preferably at least 90%, 95%, 98% or 99%, e.g. 95 %, 96 %, 97 %, 98 %, or 99 % sequence identity to the protein of any one of (i) to (iv), preferably (i) or (ii), more preferably (i).

In particular, the mature and/or active TFB2M protein may be a human mature TFB2M protein with a sequence set forth in SEQ ID NO:12 or SEQ ID NO:16, preferably SEQ ID NO:12, and/or a protein that has at least 60%, 70%, 80% or 90%, preferably at least 90%, 95%, 98% or 99%, e.g. 95 %, 96 %, 97 %, 98 %, or 99 % sequence identity to said human mature TFB2M protein. More preferably, the mature TFB2M protein has a sequence set forth in SEQ ID NO:12 or SEQ ID NO:16, preferably SEQ ID NO:12.

As used herein, the immature TFB2M protein may refer to:
(i) a human immature TFB2M protein with a sequence set forth in SEQ ID NO:10, SEQ ID NO:14 or SEQ ID NO:18, preferably SEQ ID NO:10;
(ii) a protein that is encoded by a nucleotide sequence set forth in SEQ ID NO:9, SEQ ID NO:13 or SEQ ID NO:17, preferably SEQ ID NO:9;
(iii) an immature TFB2M protein that is orthologous to the human immature TFB2M protein of (i);
(iv) a protein that is encoded by a nucleotide sequence which is orthologous to the nucleotide sequence described in (ii); and/or
(v) a protein that has at least 60%, 70%, 80% or 90%, preferably at least 90%, 95%, 98% or 99%, e.g. 95 %, 96 %, 97 %, 98 %, or 99 % sequence identity to the protein of any one of (i) to (iv), preferably (i) or (ii), more preferably (i).

In particular, the immature TFB2M protein may be a human immature TFB2M protein with a sequence set forth in SEQ ID NO:10, SEQ ID NO:14 or SEQ ID NO:18, preferably SEQ ID NO:10, and/or a protein that has at least 60%, 70%, 80% or 90%, preferably at least 90%, 95%, 98% or 99%, e.g. 95 %, 96 %, 97 %, 98 %, or 99 % sequence identity to said human immature TFB2M protein. More preferably, the immature TFB2M protein has a sequence set forth in SEQ ID NO:10, SEQ ID NO:14, or SEQ ID NO:14, preferably SEQ ID NO:10.

In particular, the immature TFB2M protein, as used herein, contains a N-terminal part that is cleaved off when the mature TFB2M protein is generated. Accordingly, the mature and/or active TFB2M protein, as used herein, does, in particular, not contain, i.e. not at the N-terminal end, a sequence corresponding to the N-terminal part of the immature TFB2M protein that is cleaved off when the mature TFB2M protein is generated. For example, said N-terminal part of the immature TFB2M protein may have the amino acid sequence set forth in positions 1 to 43 of SEQ ID NO:10 (i.e. as set forth in SEQ ID NO: 24), an amino acid sequence encoded by the nucleic acid sequence set forth in positions 1 to 129 of SEQ ID NO:9 (i.e. as set forth in SEQ ID NO: 23), or an amino acid sequence that has at least 60%, 70%, 80% or 90%, preferably at least 90%, 95%, 98% or 99%, e.g. 95 %, 96 %, 97 %, 98 %, or 99 % sequence identity to any of said amino acid sequences.

As commonly understood in the art and as also used herein and in context of the present invention, the term "sequence identity", refers to the extent to which two (nucleotide or amino acid) sequences have the same residues at the same positions in an alignment. Typically, the "sequence identity" is expressed as a percentage. Moreover, one of the two sequences may be considered as a reference sequence to which the other sequence has a sequence identity of at least n %.

The degree of sequence identity can be determined according to methods well known in the art using, preferably, suitable computer algorithms such as BLAST and/or CLUSTAL Omega. In particular, BLAST may be used in combination with CLUSTAL Omega.

When using the Clustal Omega analysis method (e.g. in combination with BLAST) to determine whether a particular sequence has a certain % sequence identity to a reference sequence default settings may be used.

Preferably, Clustal Omega (Madeira F, Park YM, Lee J, et al. The EMBL-EBI search and sequence analysis tools APIs in 2019. Nucleic Acids Research. 2019 Jul;47(W1):W636-W641. DOI: 10.1093/nar/gkz268. PMID: 30976793; PMCID: PMC6602479) is used for the comparison of amino acid sequences in context of the present invention. In the case of pairwise comparisons/alignments, the following default settings are preferably chosen: Program : clustalo; Version : 1.2.4; Input Parameters: Output guide tree: true; Output distance matrix: false; Dealign input sequences: false; mBed-like clustering guide tree: true; mBed-like clustering iteration: true; Number of iterations: 0; Maximum guide tree iterations: -1; Maximum HMM iterations: -1; Output alignment format: clustal_num; Output order: aligned; Sequence Type: protein.

Furthermore, a standard tool, preferably Clustal Omega, is used for determining the sequence identity of a nucleic acid sequence to a corresponding reference nucleic acid sequence in context of the present invention, preferably by using default settings.

The skilled person has no difficulties in identifying an orthologue of a protein (e.g. mature TFAM or immature TFAM) or a nucleotide encoding such a protein in a different species, e.g. in a different mammal such as, *inter alia,* a horse, dog, cat, cow, pig, goat, sheep, mouse, rat, guinea pig, rabbit, camel, alpaca, or monkey, for example, by interrogating well known databases such as *inter alia* Ensembl, Pubmed and/or Genome Browser.

The mature and/or active TFAM protein described herein may be contained within the mitochondrial core transcription initiation complex. Thus, the interaction of TFAM protein with TFB2M protein may occur, in particular, within the mitochondrial core transcription initiation complex.

Furthermore, the mature and/or active TFAM protein may promote the function of mitochondria, in particular (i) the transcription of mitochondrial genes, (ii) the replication of mitochondrial DNA, and/or (iii) the production of ATP, e.g., as illustrated in WO 2022/106666.

Herein, and the context of the present invention, the level of mature and/or active TFAM protein may correspond to
(i) the amount and/or concentration of mature TFAM protein, preferably the amount of mature TFAM protein,
(ii) the number of interactions of TFAM protein with at least one other mitochondrial protein (preferably TFB2M) and/or mitochondrial DNA to which the mature TFAM protein binds specifically, and/or
(iii) the ratio of the amount of mature TFAM protein over the amount of immature TFAM protein.

In particular, the number of interactions of TFAM protein with at least one other mitochondrial protein (preferably TFB2M), and/or mitochondrial DNA to which the mature TFAM protein binds specifically, is indicative of the amount of mature and/or active TFAM protein.

The amount and/or concentration of mature TFAM protein (e.g. in mitochondria) and/or the amount and/or concentration of immature TFAM protein (e.g. in the cytonucleoplasm) may be determined by routine means, e.g., as described in WO 2022/106666.

A protein to which TFAM protein binds specifically in the mitochondria may be considered a binding partner of mature and/or active TFAM protein. For example, the binding may be considered specific, when the dissociation constant (K_{d}) is 10⁻³ or smaller, preferably 10⁻⁴ or smaller, e.g. as determined by an electromobility shift assay (EMSA) or surface plasmon resonance (e.g. Biacore).

Furthermore, the mature and/or active TFAM protein may specifically bind to mitochondrial DNA in general (i.e. it may be localized and/or enriched at the mitochondrial DNA), and/or it may specifically to a certain mitochondrial DNA sequence. Binding of TFAM protein to mitochondrial DNA in general may be measured by methods known in the art, e.g. by chromatin immunoprecipitation methods.

The binding to a certain mitochondrial DNA sequence may be considered specific, when TFAM binds preferably (e.g. above background noise) to this sequence, e.g. as determined by ChIP-seq and/or EMSA. This type of binding is typically referred to as "sequence specific binding". For example, a specific mitochondrial DNA binding sequence of TFAM and/or TFB2M may be a TFAM and TFB2M consensus binding sequence, e.g., *inter alia,* AAAGATAAAATTTGAAAT (SEQ ID NO: 19) or AAAGACACCCCCCACAG (SEQ ID NO: 20), or a sequence that has at least 60%, 70%, 80%, 90% or 95% sequence identity to such an exemplary consensus sequence. In particular, the binding of TFAM protein to a protein binding partner, mitochondrial DNA in general (i.e. non-sequence specific binding), and/or a certain mitochondrial DNA sequence (i.e. sequence-specific binding) has a biological effect, e.g. it may promote the mitochondrial biogenesis as described herein. Thus, the number and/or amount of interactions of TFAM protein with at least one other mitochondrial protein and/or mitochondrial DNA to which the mature TFAM protein binds specifically (non-sequence specific and/or sequence-specific) is indicative of the level (e.g amount) of mature and/or active TFAM protein and/or the activity level of TFAM protein.

Suitable protein binding partners of TFAM include TFB2M, mitochondrial RNA polymerase, nuclear respiratory factor 1, peroxisome proliferator-activated receptor gamma coactivator 1-alpha, GA binding protein transcription factor subunit alpha, cytochrome C, mitochondrial transcription factor B1, and/or single-stranded DNA-binding protein 1. In the context of the invention, the binding partner of TFAM protein, in particular mature and/or active TFAM protein, is preferably TFB2M protein, in particular, mature and/or active TFB2M protein.

Furthermore, the amount of mature and/or active TFAM protein may correspond to the amount of mature TFAM protein normalized by the amount of TNF receptor associated protein 1 (TRAP1), in particular the amount of TRAP1 protein, in particular in the mitochondria.

Furthermore, the amount of immature TFAM protein may correspond to the amount of immature TFAM protein normalized by the amount of β-actin, in particular the amount of β-actin protein, in particular in the cytonucleoplasm.

The term "cytonucleoplasm", as used herein refers to the entire content of a cell without mitochondria, in particular to the cytoplasm (except for mitochondria) including, *inter alia,* the cytosol, endoplasmatic reticulum, golgi, lysosome, and the nucleus. Preferably, neither the cytonucleoplasm nor the mitochondria include the cell plasma membrane. The mitochondria may be separated or distinguished from the cytonucleoplasm *in* vitro and/or *in silica,* e.g. as illustrated in the appended Examples and/or by methods known in the art.

Furthermore, the level of mature and/or active TFAM protein may correspond to the ratio (R) of the amount of mature and/or active TFAM protein (M) normalized by the amount of TRAP1 protein (T) over the amount of immature TFAM protein (I) normalized by the amount of β-actin (B), as described by the formula: R = (M/T) / (I/B).

In particular, the amount of mature and/or active TFAM protein and the amount of TRAP1 protein may be determined in the mitochondria, whereas the amount of immature TFAM protein and β-actin protein may be determined in the cytonucleoplasm.

Particularly preferably herein, as further described above, the level of mature and/or active TFAM protein may correspond to the number of interactions of TFAM protein with mitochondrial transcription factor B2 (TFB2M) protein, i.e., the amount of TFAM-TFB2M complex.

In the context of the inventive methods provided herein, determining the level of mature and/or active TFAM protein may comprise quantifying the interaction of TFAM protein with at least one other mitochondrial protein (preferably TFB2M protein) and/or mitochondrial DNA to which the mature TFAM protein binds specifically.

Quantifying the interaction of TFAM protein with mitochondrial DNA may be done, e.g. by chromatin immunopreciptiation (ChIP) using an anti-TFAM antibody (or an antigen-binding fragment thereof), for example by ChIP-seq.

It is also possible to infer the level of mature and/or active TFAM protein by a functional assay, e.g. a reporter (e.g. luciferase) assay, wherein the activation of a TFAM-specific promoter and/or enhancer by mature and/or active TFAM protein leads to expression of a reporter protein (e.g. a luciferase or a fluorescent protein).

Preferably, however, determining the level of mature and/or active TFAM protein comprises quantifying the interaction of TFAM protein with at least one other mitochondrial protein to which the mature TFAM protein binds specifically, preferably TFB2M protein.

In some embodiments of the invention, the amount and/or number of interactions of TFAM with a protein binding partner (e.g. TFB2M) may be quantified by an electromobility shift assay (EMSA), Co-Immunopreciptiation (CoIP) and/or a FRET/FLIM assay.

Preferably, however, in the context of the present invention, quantifying the interaction of TFAM protein with at least one other mitochondrial protein to which the mature and/or active TFAM protein binds specifically, preferably TFB2M protein, more specifically mature and/or active TFB2M protein, may comprise the steps of
(a) contacting the sample with a pair of antigen-binding molecules,
   wherein one of said antigen-binding molecules specifically binds TFAM protein, and wherein the other of said antigen-binding molecules specifically binds said other mitochondrial protein to which the mature TFAM protein binds specifically (e.g. TFB2M protein), and
(b) generating a detectable signal when said two antigen-binding molecules are in close proximity to each other, in particular when the distance between said two antigen-binding molecules is about 100, 80, 60 or 40 nm or less, preferably about 40 nm or less, e.g. about 40 nm or about 20 nm,
thereby quantifying said interaction.

Hence, determining the level of mature and/or active TFAM protein may preferably comprise quantifying the interaction of TFAM protein with TFB2M protein, in particular the number of interactions of TFAM protein with TFB2M protein (i.e., the amount of TFAM-TFB2M complex), in the sample.

Thus, in preferred embodiments, measuring the interactions of TFAM protein with TFB2M protein, comprises the steps of
(a) contacting the sample with a pair of antigen-binding molecules, preferably antibodies,
   wherein one of said antigen-binding molecules specifically binds TFAM protein, and wherein the other of said antigen-binding molecules specifically binds TFB2M protein, and
(b) generating a detectable signal when said two binding molecules are in close proximity to each other.

Evidently, the number of interactions in a sample does not necessarily refer to all interactions physically present in the sample, but may refer to a representative subset thereof, e.g., to the interactions detected by a measurement method described herein.

In the context of the invention, e.g. in the context of quantifying the interaction of TFAM protein with at least one other mitochondrial protein to which the mature TFAM protein binds specifically, an antigen-binding molecule may be an antibody (or an antigen-binding fragment thereof), a monobody, or an aptamer, preferably an antibody or an antigen-binding fragment thereof. In principle, however, any molecule may be considered an antigen-binding molecule if it specifically binds to its target antigen (e.g. mature TFAM protein), preferably with a K_{D} of 10⁻⁸ M or smaller, and preferably does not bind to other molecules in the sample (e.g. immature TFAM protein) with such a low K_{D}. Preferably, the antigen-binding molecule, in context of the invention, is an antibody or an antigen-binding fragment thereof).

Herein and in context of the present invention, an antibody or antigen-binding fragment thereof may be, *inter alia,* a full antibody (immunoglobulin), a F(ab)-fragment, a F(ab)2-fragment, a disulfide-linked Fvs (sdFv), an anti-idiotypic (anti-Id) antibody, an epitope-binding fragment, a single chain antibody such as a single-chain variable fragment (scFv), or a nanobody / single-domain antibody. Furthermore, the antibody or fragment thereof may be, *inter alia,* a monoclonal antibody, a polyclonal antibody, a recombinantly produced antibody, a chimeric antibody, a humanized antibody, a human antibody, a fully human antibody, or a CDR-grafted antibody, or an antigen-binding fragment of any one of said antibodies.

A monobody, as used herein, refers, in particular, to a synthetic binding protein that is constructed using a fibronectin type III domain (FN3) as a molecular scaffold.

An aptamer, as used herein, refers, in particular, to an oligonucleotide or peptide molecule that binds to a specific target molecule. For example, aptamers include (i) DNA or RNA or XNA aptamers which may consist of, usually short, strands of oligonucleotides, and (ii) peptide aptamers which consist of one or more short variable peptide domains, and which may be attached at both ends to a protein scaffold.

In the context of the present invention, generating a detectable signal, may comprise the steps of
(a) generating an oligonucleotide template when the two antigen-binding molecules (e.g., an anti-TFAM antibody and an anti-TFB2M antibody) are in close proximity to each other, e.g. about 40 nm or less apart from each other, and
(b) amplifying and/or extending said oligonucleotide template, in particular wherein the amplified and/or extended oligonucleotide is labeled, preferably with a fluorophore.

For example, the oligonucleotide template may be formed by ligation of at least two oligonucleotides (e.g. proximity oligonucleotides) and may be linear or circularized (e.g. as in PLA), and/or the oligonucleotide template may be formed by a change in secondary or tertiary structure of an oligonucleotide (e.g. proximity oligonucleotide) and/or by binding of at least one other oligonucleotide to said oligonucleotide (e.g. as in ProxHCR).

Preferably, the amplified and/or extended oligonucleotide may be generated and detected at the position, where said two antigen-binding molecules are located.

A suitable fluorophore may be, *inter alia,* Alexa Fluor 488, Alexa Fluor 546, ATTO 390, ATTO 488, ATTO 565, ATTO 680, ATTO 700, Cy 3, Cy 3.5, Cy 5, Cy 5.5, Cy 7, FITC, TRITC, Texas Red, FAM, or TAMRA *etc.*

The detectable signal may be quantified by imaging, flow cytometry and/or a point-of-care device, e.g., as illustrated in the appended Examples or in WO 2022/106666.

Furthermore, each of the two antigen-binding molecules may comprise an oligonucleotide (e.g. a proximity oligonucleotide) and/or is specifically bound by a secondary (antigen-) binding molecule (e.g. secondary antibody) comprising such an oligonucleotide. In particular, the two oligonucleotides linked to the two antigen-binding molecules can form an oligonucleotide template when said two antigen-binding molecules are in close proximity to each other, as described herein. Furthermore, said oligonucleotide template can be amplified and/or extended. For example, an oligonucleotide (e.g. a proximity oligonucleotide) comprised in (e.g. attached/conjugated to) an antigen-binding molecule may have a length of about 10 bp to about 200 bp, preferably about 20 bp to about 100 bp.

In preferred embodiments, the two antigen-binding molecules refer to an antibody specifically binding TFAM protein and an antibody specifically binding TFB2M protein, as described herein.

Preferably, an antigen-binding molecule is an antibody conjugated to an oligonucleotide (e.g. proximity oligonucleotide) and/or an antigen-binding molecule is specifically bound by an antibody conjugated to an oligonucleotide (e.g. proximity oligonucleotide).

In preferred embodiments, quantifying the interaction of TFAM protein with at least one other mitochondrial protein to which the mature TFAM protein binds specifically (e.g. TFB2M) comprises performing a proximity ligation assay (PLA) and/or a proximity-dependent initiation of hybridization chain reaction (proxHCR), e.g. as described in the appended Examples or in WO 2022/106666. ProxHCR is further described in Koos (2014), Nature Communications 6, and the published patent application US20170009278. PLA is further described in Söderberg (2006), Nat Methods, 3(12) and Clausson (2015), Sci Rep 5, 12317.

In particularly preferred embodiments, the interactions of TFAM protein with TFB2M protein are measured by a proximity ligation assay (PLA) and/or a proximity-dependent initiation of hybridization chain reaction (proxHCR), preferably a proximity ligation assay (PLA).

Preferably herein, the sample is a blood sample. The sample may comprise a cell, a cell or tissue (e.g. blood) lysate, and/or a cell or tissue (e.g. blood) extract, for example, it may be a whole blood lysate. In particular, the cell comprises mitochondria. Preferably, the cell is a leucocyte, e.g., a peripheral blood mononuclear cell. Preferably herein, the tissue is blood. In particular, for determining the level of mature and/or active TFAM protein, the sample comprises mitochondria. Thus, the cell extract preferably comprises mitochondria, and preferably does not comprise the cell nucleus and cytoplasm.

In some embodiments, the level of mature and/or active TFAM protein in the mitochondria is determined (and not in the entire cell or cytonucleoplasm). This may be done, for example, directly by isolating mitochondria *in vitro* and/or *in silica* (e.g. in a microscopic image of the cell), or indirectly by measuring the interaction of TFAM protein with TFB2M protein (which only occurs in the mitochondria). For isolating mitochondria, e.g. *in silica,* mitochondrial markers may be employed which allow distinguishing mitochondria from the rest of the cell. Suitable mitochondrial markers may be, *inter alia,* TRAP1, VDAC and/or a MitoTracker^{™} (e.g., *inter alia,* Benzoxazolium, 2-[3-[5,6-dichloro-1,3-bis[[4-(chloromethyl)phenyl]methyl]-1,3-dihydro-2H-benzimidazol-2-ylidene]-1-propenyl]-3-methyl-, chloride 201860-17-5 or 1H,5H,11H,15H-Xantheno[2,3,4-ij:5,6,7-i'j']diquinolizin-18-ium, 9-[4-(chloromethyl)phenyl]-2,3,6,7,12,13,16,17-octahydro-, chloride 167095-09-2).

Thus, the level of TFAM may correspond herein and in context of the invention to the amount of mature TFAM protein in mitochondria.

The methods of the invention may further comprise measuring the level of at least one cytokine and/or chemokine (preferably at least one cytokine) in a sample (preferably a blood sample) from the subject. Preferably, the cytokine(s) and/or chemokine(s) is/are measured in the serum or plasma, more preferably the serum, of a blood sample.

The cytokines and/or chemokines are not particularly limited, and any of them may be used in context of the present invention, in particular, for predicting a muscle, tendon and/or ligament injury in a subject in combination with the level of at least one biomarker for mitochondrial resilience and/or functionality, as described herein.

For example, the cytokine(s) according to the invention may comprise at least one interleukin, at least one interferon, and/or TNF-o (Tumor necrosis factor).

In preferred embodiments, the cytokine(s) comprise(s) IL-6, IL-8, IL-10 and/or TNF-o.

A level of IL-8 below a respective threshold may be indicative of a high likelihood that a muscle, tendon and/or ligament injury will occur in the subject, and/or a level of IL-8 equal to or above a respective threshold may be indicative of a low likelihood that a muscle, tendon and/or ligament injury will occur in the subject.

Furthermore, (a) level(s) of IL-6, IL-10 and/or TNF-o below (a) respective threshold(s) may be indicative of a low likelihood that a muscle, tendon and/or ligament injury will occur in the subject, and/or (a) level(s) of IL-6, IL-10 and/or TNF-o equal to or above (a) respective threshold(s) may be indicative of a high likelihood that a muscle, tendon and/or ligament injury will occur in the subject.

The level of a cytokine or a chemokine can be measured by routine methods in the art, e.g., an immunoassay employing an antibody directed against a cytokine or chemokine such as an ELISA, flow cytometry (e.g., using antibodies on beads) or a Luminex^{®} Multiplex Assay. Preferably, the level of a cytokine or a chemokine is measured by using a Luminex^{®} Multiplex Assay, preferably as illustrated in the appended non-limiting Examples.

In some embodiments, the method of the invention comprises measuring the levels of ATP and IL-6 in a blood sample from the subject,
(i) wherein a level of ATP below a respective threshold and a level of IL-6 equal to or above a respective threshold are indicative of a high likelihood that a muscle, tendon and/or ligament injury will occur in the subject, and/or
(ii) wherein a level of ATP equal to or above a respective threshold and a level of IL-6 below a respective threshold are indicative of a low likelihood that a muscle, tendon and/or ligament injury will occur in the subject.

In some embodiments, the method of the invention comprises measuring the levels of ATP and IL-10 in a blood sample from the subject,
(i) wherein a level of ATP below a respective threshold and a level of IL-10 equal to or above a respective
   threshold are indicative of a high likelihood that a muscle, tendon and/or ligament injury will occur in the subject, and/or
(ii) wherein a level of ATP equal to or above a respective threshold and a level of IL-10 below a respective threshold are indicative of a low likelihood that a muscle, tendon and/or ligament injury will occur in the subject.

In some embodiments, the method of the invention comprises measuring the levels of ATP and TNF-o in a blood sample from the subject,
(i) wherein a level of ATP below a respective threshold and a level of TNF-o equal to or above a respective threshold are indicative of a high likelihood that a muscle, tendon and/or ligament injury will occur in the subject, and/or
(ii) wherein a level of ATP equal to or above a respective threshold and a level of TNF-o below a respective threshold are indicative of a low likelihood that a muscle, tendon and/or ligament injury will occur in the subject.

In some embodiments, the method of the invention comprises measuring the levels of ATP and IL-8 in a blood sample from the subject,
(i) wherein levels of ATP and IL-8 below respective thresholds are indicative of a high likelihood that a muscle, tendon and/or ligament injury will occur in the subject, and/or
(ii) wherein levels of ATP and IL-8 equal to or above respective thresholds are indicative of a low likelihood that a muscle, tendon and/or ligament injury will occur in the subject.

In some embodiments, the method of the invention comprises measuring the levels of TFAM and IL-6 in a blood sample from the subject,
(i) wherein a level of TFAM below a respective threshold and a level of IL-6 equal to or above a respective threshold are indicative of a high likelihood that a muscle, tendon and/or ligament injury will occur in the subject, and/or
(ii) wherein a level of TFAM equal to or above a respective threshold and a level of IL-6 below a respective threshold are indicative of a low likelihood that a muscle, tendon and/or ligament injury will occur in the subject.

In some embodiments, the method of the invention comprises measuring the levels of TFAM and IL-10 in a blood sample from the subject,
(i) wherein a level of TFAM below a respective threshold and a level of IL-10 equal to or above a respective threshold are indicative of a high likelihood that a muscle, tendon and/or ligament injury will occur in the subject, and/or
(ii) wherein a level of TFAM equal to or above a respective threshold and a level of IL-10 below a respective threshold are indicative of a low likelihood that a muscle, tendon and/or ligament injury will occur in the subject.

In some embodiments, the method of the invention comprises measuring the levels of TFAM and TNF-o in a blood sample from the subject,
(i) wherein a level of TFAM below a respective threshold and a level of TNF-o equal to or above a respective threshold are indicative of a high likelihood that a muscle, tendon and/or ligament injury will occur in the subject, and/or
(ii) wherein a level of TFAM equal to or above a respective threshold and a level of TNF-o below a respective threshold are indicative of a low likelihood that a muscle, tendon and/or ligament injury will occur in the subject.

In some embodiments, the method of the invention comprises measuring the levels of TFAM and IL-8 in a blood sample from the subject,
(i) wherein levels of TFAM and IL-8 below respective thresholds are indicative of a high likelihood that a muscle, tendon and/or ligament injury will occur in the subject, and/or
(ii) wherein levels of TFAM and IL-8 equal to or above respective thresholds are indicative of a low likelihood that a muscle, tendon and/or ligament injury will occur in the subject.

In some embodiments, the method of the invention comprises measuring the levels of ATP and TFAM and at least one cytokine selected from IL-6, IL-8, IL-10 and TNF-o, in a blood sample from the subject,
(i) wherein levels of ATP and TFAM (and optionally IL-8) below respective thresholds, and optionally (a) level(s) of IL-6, IL-10 and/or TNF-o equal to or above (a) respective threshold(s), are indicative of a high likelihood that a muscle, tendon and/or ligament injury will occur in the subject, and/or
(ii) wherein levels of ATP and TFAM (an optionally IL-8) equal to or above respective thresholds, and optionally (a) level(s) of IL-6, IL-10 and/or TNF-o below (a) respective threshold(s), are indicative of a low likelihood that a muscle, tendon and/or ligament injury will occur in the subject.

Furthermore, the methods of the invention may comprise measuring the level of at least one reactive oxygen species (ROS), or oxidative stress in general, in a sample (preferably a blood sample) from the subject. Preferably, the oxidative stress and/or ROS is/are measured in the serum or plasma, more preferably the plasma, of a blood sample. In particular, (a) ROS level(s) equal to or above respective (a) threshold(s) may be indicative of a high likelihood that a muscle, tendon and/or ligament injury will occur in the subject, and/or (a) ROS level(s) below (a) respective threshold(s) may be indicative of a low likelihood that a muscle, tendon and/or ligament injury will occur in the subject.

The level of oxidative stress and/or ROS can be measured by routine methods in the art, e.g., by using an indicator dye such as, *inter alia,* H2DCFDA or CM-H2DCFDA (Thermo Fisher Scientific/Invitrogen, Freiburg, Germany), fluorescent substances that show a shift in the fluorescence spectrum upon oxidation such as roGFP2, FRET probes such as HyPer, or a Seahorse system by Agilent.

In context of the present invention, the level(s) of the biomarker(s) for mitochondrial resilience and/or functionality and/or of the cytokine(s) described herein is/are, preferably, measured before a physical exercise of the subject and/or after a physical exercise of the subject, more preferably at least after the physical exercise, even more preferably before and after the physical exercise.

A level of a molecule (e.g., a biomarker for mitochondrial resilience and/or functionality or a cytokine) or of a cell status (e.g. oxidative stress) measured before a physical exercise of a subject, is denoted herein and in context of the present invention, also as "baseline level". Preferably herein, the baseline level is measured about 1 minute to about 12 hours, e.g., about 1 minute, about 5 minutes, about 10 minutes, about 15 minutes, about 20 minutes, about 30 minutes, about 45 minutes, about 1 hour, about 2 hours, about 3 hours, about 4 hours, about 5 hours, about 6 hours, about 8 hours, about 10 hours or about 12 hours, before a physical exercise. In some preferred embodiments, the baseline level is measured at most about one hour, e.g., about 1 minute, about 5 minutes, about 10 minutes, about 15 minutes, about 20 minutes, about 30 minutes, about 45 minutes or about 1 hour, before a physical exercise.

A level of a molecule (e.g., a biomarker for mitochondrial resilience and/or functionality or a cytokine) or of a cell status (e.g. oxidative stress) measured after a physical exercise of a subject, is denoted herein and in context of the present invention, also as "stress level". Preferably herein, the stress level is measured about 1 minute to about 12 hours, e.g., about 1 minute, about 5 minutes, about 10 minutes, about 15 minutes, about 20 minutes, about 30 minutes, about 45 minutes, about 1 hour, about 2 hours, about 3 hours, about 4 hours, about 5 hours, about 6 hours, about 8 hours, about 10 hours or about 12 hours, after a physical exercise. In some preferred embodiments, the stress level is measured at most about one hour, e.g., about 1 minute, about 5 minutes, about 10 minutes, about 15 minutes, about 20 minutes, about 30 minutes, about 45 minutes or about 1 hour, after a physical exercise.

In some preferred embodiments, the level(s) of the biomarker(s) for mitochondrial resilience and/or functionality corresponds to the stress level(s) of said biomarker(s).

In further preferred embodiments, the level(s) of the biomarker(s) for mitochondrial resilience and/or functionality and/or of the cytokine(s) described herein in a subject (preferably in blood sample thereof) is/are, preferably, measured over time, more preferably, before and after a physical exercise.

The baseline level and stress level (e.g. of a biomarker for mitochondrial resilience and/or functionality) may be specific for an individual, or a certain physical exercise of an individual. Hence, a baseline level may serve as an individual reference level for a subject (or for a certain physical exercise of the subject). More specifically, the baseline level can be used for normalizing the level (in particular the stress level) of a biomarker for mitochondrial resilience and/or functionality (e.g., TFAM or ATP).

In particularly preferred embodiments, the level of a biomarker for mitochondrial resilience and/or functionality (e.g. the level of ATP or the level of TFAM) corresponds, preferably, to a normalized level (in particular a normalized stress level) that is calculated by normalizing the stress level(s) of said biomarker(s) to the respective baseline level(s). Preferably herein, a normalized level (in particular a normalized stress level) is calculated according to the following formula: (stress level - baseline level) / baseline level, i.e., (stress level minus baseline level) divided by the baseline level.

Thus, a normalized level of a biomarker for mitochondrial resilience and/or functionality (e.g. TFAM or ATP) according to the invention may be also considered herein as "normalized stress level".

The stress level(s), in particular the normalized stress levels, of at least one biomarker for mitochondrial resilience and/or functionality (e.g. TFAM and/or ATP) according to the invention is/are, in particular, indicative of the extent of mitochondrial resilience and/or functionality, more specifically, mitochondrial repair, mitochondrial biogenesis, mitochondrial transcription and/or cellular respiration, during and/or after a physical exercise, as described herein.

In preferred embodiments of the invention, the TFAM baseline level and the TFAM stress level are measured in a subject. Preferably herein, the TFAM level (which is used for predicting a sports-associated injury such as a muscle, tendon and/or ligament injury in a subject) corresponds to a normalized TFAM level, in particular a normalized stress level. Preferably herein, the normalized TFAM (stress) level is calculated by the following formula: (TFAM stress level - TFAM baseline level) / TFAM baseline level).

In particularly preferred embodiments, the TFAM level (which is used for predicting a sports-associated injury such as a muscle, tendon and/or ligament injury in a subject) corresponds to the normalized stress level of active and/or mature TFAM protein as described herein, and/or the ATP level (which is used for predicting a sports-associated injury such as a muscle, tendon and/or ligament injury in a subject) corresponds to the normalized stress level of ATP.

The use of a normalized level (in particular a normalized stress level) of a biomarker for mitochondrial resilience and/or functionality (e.g., an ATP level or a TFAM level) may provide an absolute threshold, wherein a level below this threshold is indicative of a high likelihood that a muscle, tendon and/or ligament injury will occur and/or a level equal to or above this threshold is indicative of a low likelihood that a muscle, tendon and/or ligament injury will occur.

In some embodiments, the threshold of the normalized TFAM (stress) level (which may be used for predicting a sports injury such as a muscle, tendon and/or ligament injury in a subject) is in the range of about 0 to 0.4, preferably about 0.1 to 0.3. More preferably, the threshold of said normalized TFAM level is about 0.2.

In further preferred embodiments of the invention, the ATP baseline level and the ATP stress level are measured in a subject. Preferably herein, the ATP level (which is used for predicting a sports-associated injury such as a muscle, tendon and/or ligament injury in a subject) corresponds to a normalized ATP level. Preferably herein, the normalized ATP level is calculated by the following formula: (ATP stress level - ATP baseline level) / ATP baseline level).

In some embodiments, the threshold of the normalized ATP level (which may be used for predicting a sports injury such as a muscle, tendon and/or ligament injury in a subject) is in the range of about 0.1 to 1.7, preferably about 0.6 to 1.1. More preferably, the threshold of said normalized ATP level is about 0.9.

Furthermore, the level(s) of the cytokine(s), e.g., of TNF-o, as described herein, may be measured at least after a physical exercise of the subject (i.e., to determine the stress level(s)). Thus, the level(s) of the cytokines(s) may correspond, herein, to the stress level(s) of said cytokine(s).

Alternatively, the level(s) of the cytokine(s), e.g., of IL-6, IL-8 and/or IL-10, as described herein, may be measured multiple times, preferably before a physical exercise of the subject (i.e., to determine the baseline level(s)) and after the physical exercise of the subject (i.e., to determine the stress level(s)). In particular, the level(s) of the cytokines(s) may correspond, herein, also to the maximum level measured, e.g., the maximum level selected from the respective baseline and stress levels.

In some embodiments, the threshold of the IL-8 level is about 30 pg/ml, preferably about 27 pg/ml; the threshold of the IL-6 level is about 1 pg/ml, preferably about 1.3 pg/ml; the threshold of the IL-10 level is about 1 pg/ml, preferably about 1.4 pg/ml; and/or the threshold of the TNF-o level is about 4 pg/ml, preferably about 3.7 pg/ml.

The skilled person can readily adjust the threshold of level (e.g., normalized stress level) of a biomarker for mitochondrial resilience and/or functionality (e.g., an ATP level or a TFAM level) or the threshold of a level of a cytokine (e.g., IL-6, IL-8, IL-10 or TNF-a), if necessary, e.g., when a different measurement method is used than the one(s) employed in the appended illustrative non-limiting Examples.

Suitable thresholds for the (normalized or not normalized) level(s) of at least one biomarker for mitochondrial resilience and/or functionality, for at least one cytokine or chemokine, and/or for oxidative stress / at least one ROS, as described herein, can be also easily determined by the skilled person *de novo.* For example, a threshold (i.e., a threshold level for a specific molecule such as the TFAM-TFB2M complex, ATP or a specific cytokine such as IL-6, IL-8, IL-10 or TNF-o) may be determined by analyzing the level of the molecule in samples from a plurality of reference subjects (e.g., athletes), e.g., after a physical exercise (e.g. an athletic training), or before and after a physical exercise, wherein it is known whether the subjects later experienced a sports-associated injury such as a muscle, tendon and/or ligament injury. Specifically, suitable thresholds may be readily identified by using a computational model, e.g., machine learning, wherein training data (e.g., from reference subjects) are provided.

Generally, the methods of the invention may be, at least partly, performed on a computer (e.g., *inter alia,* a PC, notebook, MAC, smartphone or any specialized computer device), and thus may be considered as computer-implemented methods or computer-assisted methods.

In some preferred embodiments, a score (e.g., a "sports score") is calculated based on the levels of at least two of the biomarkers for mitochondrial resilience and/or functionality, preferably TFAM and ATP, as described herein, and optionally the level(s) of at least one of said cytokine(s), preferably IL-6, IL-8, IL-10 and/or TNF-o, as described herein.

In further preferred embodiments, a score (e.g., a "sports score") is calculated based on the level(s) of at least one of the biomarkers for mitochondrial resilience and/or functionality, preferably TFAM and/or ATP, as described herein, and the level(s) of at least one of the cytokine(s) or chemokine(s), preferably IL-6, IL-8, IL-10 and/or TNF-a, as described herein.

In particular, herein, (a) level(s) of TFAM, ATP and/or IL-8 below the respective threshold(s) positively add(s) to the score, and/or (a) level(s) of IL-6, IL-10 and/or TNF-o equal to or higher than the respective threshold(s) positively add(s) to the score. As an illustrative example, a level of TFAM (e.g., a normalized stress level of active and/or mature TFAM protein) below the respective threshold may add the value of 1 to the score, a level of ATP (e.g., a normalized stress level of ATP) above the respective threshold may add the value of 0 (i.e nothing) to the score, and a level of IL-6 above the respective threshold may add the value 1 to the score.

Herein, a score below a respective threshold is, in particular, indicative of a low likelihood that a muscle, tendon and/or ligament injury will occur in the subject, and/or a score equal to or above a respective threshold is, in particular, indicative of a high likelihood that a muscle, tendon and/or ligament injury will occur in the subject.

In context of the present invention, a physical exercise is not particularly limited and may comprise, *inter alia,* an athletic training, and/or a (physical) rehabilitation exercise.

An athletic training, as used herein, may comprise, e.g., *inter alia,* a warm-up, running, stretching, jumping, swimming, rowing, cardio training, sit-ups, push-ups, climbing, cycling, a ball game including kicking, dribbling, throwing or tossing a ball, etc. and/or any physical exercise that is associated with or part of any kinds of (physical) sports such as, *inter alia,* football (soccer), American football, rugby, basketball, handball, baseball, (ice) hockey, tennis, cycling/biking, skating, boxing, wrestling, riding, swimming, track and field, rowing, climbing, or motor sports etc.

In some preferred embodiments, the physical exercise is a professional athletic training or a part thereof, performed, preferably, by a professional athlete or an elite athlete.

A rehabilitation exercise, as used herein, is, in particular, a physical rehabilitation exercise which may comprise, e.g., *inter alia,* range of motion exercises (passive and/or active), isometric exercises, isotonic exercises, resistance exercises, walking, swimming, cycling, water exercises or ladder drills.

In the context of the present invention, the subject (or the patient) may be a mammal such as, *inter alia,* a human, a horse, dog, cat, cow, pig, goat, sheep, mouse, rat, guinea pig, rabbit, camel, alpaca, or monkey. Preferably herein, and in the context of the present invention, the subject (or the patient) is a human.

In some preferred embodiments of the invention, the subject is an athlete.

The term "athlete", as used herein and in context of the invention, refers to any subject that regularly or occasionally performs an athletic training, for example, in form of elite sports, professional sports or amateur sports. Preferably herein, the athlete is a professional athlete and/or elite athlete.

A professional athlete, as used herein, makes, at least, part of his/her income by performing a specific sport. An elite athlete, as used herein, is typically a professional athlete, which performs a certain sport at a high or the highest level. As an illustrative Example, an elite soccer player may play in one of the top divisions of a country such as the first or second Bundesliga or the premier league and/or participate in international competitions like the World Cup or Champions League.

In context of the present invention, an athlete may be, e.g., a football (soccer) player, an American football player, a rugby player, a basketball player, a handball player, a baseball player, an (ice) hockey player, a tennis player, a cyclist, a skater, a boxer, a wrestler, a (horse) rider, a swimmer, a track and field athlete, a rower, a climber, or a motorcycle rider or formula 1 driver etc.

In preferred embodiments, the athlete is a player in a team sport such as, *inter alia,* football (soccer), American football, rugby, basketball, handball, baseball or (ice) hockey. Preferably said athlete is a professional and/or elite athlete.

In particularly preferred embodiments, the athlete is a football (soccer player), preferably a professional and/or elite soccer player.

In some preferred embodiments of the invention, the subject is undergoing or is scheduled for rehabilitation. In particular in context of these embodiments, the subject may a patient.

Herein and in context of the invention, a patient may be, for example, *inter alia,* a patient that had or has a stroke, a heart attack, sepsis, long COVID, or a muscle, tendon and/or ligament injury. Specifically, the patient may be undergoing or is scheduled for rehabilitation, for example, due to a stroke, a heart attack, sepsis, long COVID, or a muscle, tendon and/or ligament injury.

A patient that is undergoing or scheduled for rehabilitation may be also an athlete as described herein, that had or has, e.g., a muscle, tendon and/or ligament injury.

Herein and context of the invention, a muscle injury may comprise, in particular, an injury of at least one skeletal muscle such as, *inter alia, M. Quadriceps Femoris, M. Biceps Femoris, M. Tibialis anterior, M. Fibularis longus, M. Fibularis brevis, M. Soleus* and/or *M. Gastrocnemius.*

Herein and context of the invention, a ligament injury may comprise, in particular, ligament overstretching that may range from minor damage to a complete tear; wrist and/or ankle sprains; and/or Anterior Cruciate Ligament (ACL) injuries such as ACL tears.

Herein and in context of the invention, a tendon injury may comprise, in particular, Achilles tendonitis, patellar tendonitis (Jumper's Knee), and/or lateral epicondylitis (Tennis Elbow).

In some preferred embodiments, the injury comprises or consists of a muscle injury. Specifically, the muscle injury may be associated with a reduced muscle strength.

Preferably herein and in context of the invention, the likelihood that a muscle, tendon and/or ligament injury will occur in a subject is the likelihood that a muscle, tendon and/or ligament injury will occur in the subject within the next 14, 10 or 7 days, more preferably within the next 10 days. In particular, the time period (e.g. 14, 10 or 7 days) is calculated from the measurement of the level (preferably the stress level) of at least one biomarker for mitochondrial resilience and/or functionality (e.g. TFAM and/or ATP), and optionally the level(s) (preferably the stress level(s)) of at least one cytokine or chemokine, and/or at least one ROS or oxidative stress, as described herein.

In context of the invention, a high likelihood that a muscle, tendon and/or ligament injury will occur in the subject may further indicates that the subject is to perform no or only an easy physical exercise (such as, *inter alia,* isometric training, walking, swimming, or cycling with low resistance), and/or that the subject is to discontinue or reduce a physical exercise, as described herein.

Furthermore, in context of the invention, a low likelihood that a muscle, tendon and/or ligament injury will occur in the subject may further indicate that the subject can perform a physical exercise, preferably a strenuous physical exercise (such as, *inter alia,* sprint intervals, jumps (e.g. Box jumps), circuit trainings, Tabata Training (e.g. with squats, push- ups or cycling), Bench press, Hill sprints, or training games (e.g. in a team sports such as football or basketball)), and/or that the subject can continue or extend a physical exercise, as described herein.

In some embodiments, a high likelihood that a muscle, tendon and/or ligament injury will occur in an athlete further indicates that the athlete is to perform no or only an easy athletic training, and/or that the athlete is to discontinue or reduce an athletic training, and/or wherein a low likelihood that a muscle, tendon and/or ligament injury will occur in an athlete further indicates that the athlete can perform an athletic training, preferably a strenuous athletic training, and/or that the athlete can continue or extend an athletic training.

In some embodiments, a high likelihood that a muscle, tendon and/or ligament injury will occur in a subject that is undergoing or scheduled for rehabilitation further indicates that the subject is to perform no or only an easy (physical) rehabilitation exercise, and/or that the subject is to discontinue or reduce a (physical) rehabilitation exercise, and/or
wherein a low likelihood that a muscle, tendon and/or ligament injury will occur in a subject that is undergoing or scheduled for rehabilitation further indicates that the subject can perform a (physical) rehabilitation exercise, preferably a strenuous (physical) rehabilitation exercise, and/or that the subject can continue or extend a (physical) rehabilitation exercise.

Thus, the predictive method of the invention is useful to prevent a muscle, tendon and/or ligament injury in a subject, for example, an athlete or a subject/patient that is undergoing or scheduled for rehabilitation

In context of the invention, a muscle, tendon and/or ligament injury may be prevented by indicating to the subject to perform no or only an easy physical exercise, and/or to discontinue or reduce a physical exercise when there is a high likelihood that a muscle, tendon and/or ligament injury will occur.

In some embodiments, the method of invention comprises a step of determining the likelihood that a sports-associated injury such as a muscle, tendon and/or ligament injury will occur in the subject based on the level of at least one biomarker for mitochondrial resilience and/or functionality (e.g. TFAM and/or ATP), and optionally at least one cytokine (e.g., IL-6, IL-8, IL-10 and/or TNF-a), in at least one blood sample from the subject, as described herein.

Furthermore, the method of the invention may comprise a step of advising a subject to perform no or only an easy physical exercise, and/or to discontinue or reduce a physical exercise when there is a high likelihood that a muscle, tendon and/or ligament injury will occur, as described herein.

The present invention also relates to a method of predicting the development of the performance and/or strength of a subject, said method comprising measuring the level of at least one biomarker for mitochondrial resilience and/or functionality in a sample (in particular a blood sample) from the subject,
(i) wherein (a) level(s) of said biomarker(s) outside of (a) respective corridor(s) is/are predictive of no or a low increase of the performance and/or strength of the subject, and/or
(ii) wherein (a) level(s) of said biomarker(s) within (a) respective corridor(s) is/are predictive of a strong increase of the performance and/or strength of the subject.

Preferably, the subject is performing or has performed a physical exercise, preferably an athletic training.

Furthermore, the present invention relates to a method of determining the performance level and/or strength level of a subject, said method comprising measuring the level of at least one biomarker for mitochondrial resilience and/or functionality in a sample (in particular a blood sample) from the subject,
(i) wherein (a) level(s) of said biomarker(s) outside of (a) respective corridor(s) is/are indicative of a low performance and/or strength level of the subject, and/or
(ii) wherein (a) level(s) of said biomarker(s) within (a) respective corridor(s) is/are indicative of a high performance and/or strength level of the subject.

The term "performance", as used herein, refers, in particular, to the physical performance of a subject, preferably an athlete. Preferably, herein, the performance corresponds to or correlates with Distance and Speed Metrics (e.g., total distance, average velocity and/or maximal velocity), Load and Exertion Metrics (e.g., total load and/or heart rate exertion), Acceleration and Deceleration Metrics (e.g., maximum effort acceleration and/or maximum effort deceleration), Metabolic Power Metrics (e.g., total effort in metabolic power bands), and/or Jump and Impact Metrics (e.g., jump count per minute and/or total jumps).

The term "strength", as used herein, refers, in particular, to the muscle strength of a subject, preferably an athlete. Preferably, herein, the strength correlates with metrics for muscle strength (e.g. muscle circumference, grip strength and/or maximal weight lifted).

As mentioned above, pairs of terms such as "low" and "high", or "low" and "strong", are understood for the skilled artisan as being relative to each other.

For example, a condition (e.g., a certain level of a biomarker) that is predictive of a strong increase of the performance and/or strength of a subject means that under this condition the performance/strength will increase more as compared to a condition that is predictive of a low increase of the performance and/or strength. Accordingly, in this example, a condition (e.g., a certain level of a biomarker) that is predictive of a low increase of the performance and/or strength of a subject means that under this condition the performance/strength will increase less as compared to a condition that is predictive of a strong increase of the performance and/or strength.

The person skilled in the art has no difficulties in making proper comparisons. In particular, the person skilled in the art understands that conditions should be comparable to each other. For example, the period in which the performance and/or strength is predicted should be constant across conditions.

In another example, a condition (e.g., a certain level of a biomarker) that is indicative of a high performance and/or strength level of a subject means that under this condition the performance/strength level is higher as compared to a condition that is indicative of a low performance and/or strength level. Accordingly, in this example, a condition (e.g., a certain level of a biomarker) that is indicative of a low performance and/or strength level of a subject means that under this condition the performance/strength level is lower as compared to a condition that is indicative of a high performance and/or strength level.

Herein, a corridor has two boundaries, i.e., a lower boundary and an upper boundary, wherein the lower boundary corresponds to a lower real number than the upper boundary. For example, when the corridor is from 0.2 to 0.8, the lower boundary is 0.2 and the upper boundary is 0.8. As another example, when the corridor is from -0.5 to 0.1, the lower boundary is -0.5 and the upper boundary is 0.1.

The lower boundary of a suitable corridor of the level of a biomarker for mitochondrial resilience and/or functionality may correspond to the respective threshold of the biomarker that is used for predicting a muscle, tendon and/or ligament injury in a subject, as described herein. Thus, a level of said biomarker below the lower boundary of the corridor may be indicative of a high likelihood that a muscle, tendon and/or ligament injury will occur in the subject, and/or a level of said biomarker equal to or above the lower boundary of the corridor may be indicative of a low likelihood that a muscle, tendon and/or ligament injury will occur in the subject, as described herein.

Herein, a level of a biomarker for mitochondrial resilience and/or functionality below the lower boundary of the corridor may further indicate that the subject is to perform no or only an easy physical exercise, and/or that the subject is to discontinue or reduce a physical exercise, and/or a level of said biomarker above the upper boundary of the corridor may further indicate that the subject is to perform a strenuous physical exercise, and/or that the subject is to extend a physical exercise, as described herein. A level of a biomarker for mitochondrial resilience and/or functionality within the corridor may further indicate that the subject is to continue a physical exercise, as described herein.

Specifically, when the subject is an athlete, a level of a biomarker for mitochondrial resilience and/or functionality below the lower boundary of the corridor may further indicate that the athlete is to perform no or only an easy athletic training, and/or that the athlete is to discontinue or reduce an athletic training, and/or a level of said biomarker above the upper boundary of the corridor may further indicate that the athlete is to perform a strenuous athletic training, and/or that the athlete is to extend an athletic training, as described herein. A level of said biomarker within the corridor may further indicate that the athlete is to continue an athletic training, as described herein.

Furthermore, the method of predicting the development of the performance and/or strength of a subject or the method of determining the performance level and/or strength level of a subject may comprise measuring the level of at least one cytokine in a sample (in particular a blood sample) from said subject, as described herein. Said method(s) may also comprise measuring the oxidative stress and/or ROS as described herein.

As regards the biomarker(s) for mitochondrial resilience and/or functionality and the cytokines, and measuring the level(s) thereof, the physical exercise and the measurement time-points, the sample, and the subject, the same applies *mutatis mutandis,* as described herein in context of the inventive method of predicting a muscle, tendon and/or ligament injury in a subject, to the inventive method of predicting the development of the performance and/or strength of a subject and the method of determining the performance level and/or strength level of a subject. Furthermore, the physical exercise is, preferably, an athletic training and/or the subject is, preferably, an athlete, as described herein.

Furthermore, e.g., in context of the inventive method of predicting the development of the performance and/or strength of a subject, (a) level(s) of said cytokine(s) outside of (a) respective corridor(s) may be predictive of no or a low increase of the performance and/or strength of the subject, and/or (a) level(s) of said cytokine(s) within (a) respective corridor(s) may be predictive of a strong increase of the performance and/or strength of the subject.

Furthermore, e.g., in context of the inventive method of determining the performance level and/or strength level of a subject, (a) level(s) of said cytokine(s) outside of (a) respective corridor(s) may be indicative of a low performance and/or strength level of the subject, and/or (a) level(s) of said cytokine(s) within (a) respective corridor(s) may be indicative of a high performance and/or strength level of the subject.

Herein, a level of IL-8 below the lower boundary of the corridor may be further indicative of a high likelihood that a muscle, tendon and/or ligament injury will occur in the subject, and/or a level of said biomarker equal to or above the lower boundary of the corridor may be further indicative of a low likelihood that a muscle, tendon and/or ligament injury will occur in the subject, as described herein.

A level of IL-8 below the lower boundary of the corridor may further indicate that the subject is to perform no or only an easy physical exercise, and/or that the subject is to discontinue or reduce a physical exercise, and/or a level of IL-8 above the upper boundary of the corridor may further indicate that the subject is to perform a strenuous physical exercise, and/or that the subject is to extend a physical exercise, as described herein. A level of IL-8 within the corridor may further indicate that the subject is to continue a physical exercise, as described herein.

Specifically, when the subject is an athlete, a level of IL-8 below the lower boundary of the corridor may further indicate that the athlete is to perform no or only an easy athletic training, and/or that the athlete is to discontinue or reduce an athletic training, and/or a level of IL-8 above the upper boundary of the corridor may further indicate that the athlete is to perform a strenuous athletic training, and/or that the athlete is to extend an athletic training, as described herein. A level of IL-8 within the corridor may further indicate that the athlete is to continue an athletic training, as described herein.

Furthermore, (a) level(s) of IL-6, IL-10 and/or TNF-o below (a) respective upper boundary/(ies) of the corridor may be indicative of a low likelihood that a muscle, tendon and/or ligament injury will occur in the subject, and/or (a) level(s) of IL-6, IL-10 and/or TNF-o equal to or above (a) respective upper boundary/(ies) of the corridor may be indicative of a high likelihood that a muscle, tendon and/or ligament injury will occur in the subject.

A level of IL-6, IL-10 or TNF-α equal to or above a respective upper boundary of the corridor may further indicate that the subject is to perform no or only an easy physical exercise, and/or that the subject is to discontinue or reduce a physical exercise, and/or a level of IL-6, IL-10 or TNF-o below a respective lower boundary of the corridor may further indicate that the subject is to perform a strenuous physical exercise, and/or that the subject is to extend a physical exercise, as described herein. A level of IL-6, IL-10 or TNF-o within the respective corridor may further indicate that the subject is to continue a physical exercise, as described herein.

Specifically, when the subject is an athlete, a level of IL-6, IL-10 or TNF-o equal to or above a respective upper boundary of the corridor may further indicate that the athlete is to perform no or only an easy athletic training, and/or that the athlete is to discontinue or reduce an athletic training, and/or a level of IL-6, IL-10 or TNF-o below a respective lower boundary of the corridor may further indicate that the athlete is to perform a strenuous athletic training, and/or that the athlete is to extend an athletic training, as described herein. A level of IL-6, IL-10 or TNF-α within the respective corridor may further indicate the athlete is to continue an athletic training, as described herein.

Furthermore, the method of predicting the development of the performance and/or strength of a subject or the method of determining the performance level and/or strength level of a subject may comprise measuring the oxidative stress and/or ROS, as described herein.

Furthermore, e.g., in context of the inventive method of predicting the development of the performance and/or strength of a subject, (a) ROS level(s) outside of (a) respective corridor(s) may be predictive of no or a low increase of the performance and/or strength of the subject, and/or (a) ROS level(s) within (a) respective corridor(s) may be predictive of a strong increase of the performance and/or strength of the subject.

Furthermore, e.g., in context of the method of determining the performance level and/or strength level of a subject, (a) ROS level(s) outside of (a) respective corridor(s) may be indicative of a low performance and/or strength level of the subject, and/or (a) ROS level(s) within (a) respective corridor(s) may be indicative of a high performance and/or strength level of the subject.

Suitable corridors for the (normalized or not normalized) level(s) of at least one biomarker for mitochondrial resilience and/or functionality, for at least one cytokine or chemokine, and/or for oxidative stress / at least one ROS, as described herein, can be determined by the skilled person *de novo.* For example, a corridor (limited by upper and lower boundary/threshold levels for a specific molecule such as the TFAM-TFB2M complex, ATP or a specific cytokine such as IL-6, IL-8, IL-10 or TNF-o) may be determined by analyzing the level of the molecule in samples from a plurality of reference subjects (e.g., athletes), e.g., after a physical exercise (e.g. an athletic training), or before and after a physical exercise, wherein the present and/or future performance and/or strength levels of the subjects are known. Specifically, suitable corridors may be readily identified by using a computational model, e.g., machine learning, wherein training data (e.g., from reference subjects) are provided.

In a further aspect, the present invention relates to a kit comprising at least one means for measuring the level(s) of at least one biomarker for mitochondrial resilience and/or functionality (e.g., TFAM and/or ATP), and optionally of at least one cytokine (e.g., IL-6, IL-8, IL-10 and/or TNF-a), in a blood sample, as described herein.

In preferred embodiments, the kit comprises an antigen-binding molecule, preferably an antibody, specifically binding TFAM protein, and an antigen-binding molecule, preferably an antibody, specifically binding TFB2M protein, as described herein.

In some embodiments, the kit comprises luciferin and/or a luciferase, preferably, wherein the luciferin is D-luciferin.

Furthermore, the kit of the invention may comprise an antigen-binding molecule, preferably an antibody, specifically binding IL-6, an antigen-binding molecule, preferably an antibody, specifically binding IL-8, an antigen-binding molecule, preferably an antibody, specifically binding IL-10, and/or an antigen-binding molecule, preferably an antibody, specifically binding TNF-o, as described herein.

In some preferred embodiments, the kit comprises an antibody specifically binding TFAM, and an antibody binding at least one cytokine, preferably, selected from IL-6, IL-8, IL-10 and TNF-o. Preferably, said kit further comprises an antibody specifically binding TFB2M.

The kit of the invention may be used for measuring the level of at least one biomarker for mitochondrial resilience and/or functionality, and optionally of at least one cytokine, in a blood sample, in particular, as described herein.

In preferred embodiments, the kit of the invention is used a method of the invention, e.g., a method of predicting a muscle, tendon and/or ligament injury in a subject, a method of predicting the development of the performance and/or strength of a subject, a method of determining the performance level and/or strength level of a subject, and/or a method of detecting an abnormal stress level of a biomarker for mitochondrial resilience and/or functionality in a blood sample from a subject, as described herein.

Preferably, the kit of the invention further comprising a leaflet or brochure with instructions for carrying out a method according to the invention.

Furthermore, the present invention relates to the use of the inventive kit provided herein for measuring the level of at least one biomarker for mitochondrial resilience and/or functionality, and optionally of at least one cytokine, in a blood sample, as described herein.

Additionally, the present invention relates to a method of detecting an abnormal stress level of a biomarker for mitochondrial resilience and/or functionality in a blood sample from a subject, wherein said method comprises
(a) measuring the level of at least one biomarker for mitochondrial resilience and/or functionality in a blood sample from the subject after a physical exercise of the subject, and
(b) determining whether the blood sample is abnormal, wherein the blood sample is determined to be abnormal if the level of at least one biomarker for mitochondrial resilience and/or functionality is less than 200% higher, less than 150% higher, less than 100% higher (i.e. less than double), less than about 80% higher, less than about 60% higher, less than about 40% higher, less than about 20% higher, similar or lower than the level(s) determined for a blood sample that has been taken from the subject before the physical exercise.

The blood sample may be also determined to be abnormal if the normalized stress level of at least one biomarker for mitochondrial resilience and/or functionality, as described herein, is less than about 2, less than about 1.5, less than about 1, less than about 0.8, less than about 0.6, less than about 0.4, less than about 0.2, about 0, or less than about 0, e.g. in the range of about 0 to about 0.4 (e.g. for TFAM) or in the range of about 0.1 to 1.7 (e.g. for ATP).

In case the biomarker for mitochondrial resilience and/or functionality is TFAM (esp. active and/or mature TFAM protein), as described herein, the blood sample is, preferably, determined to be abnormal if the level of TFAM is less than about 40% higher, less than about 20% higher, less than about 10% higher or than the TFAM level determined for a blood sample that has been taken from the subject before the physical exercise.

Thus, blood sample may be also determined to be abnormal if the normalized stress level of TFAM, as described herein, is, preferably, less than 0.4, less than 0.2, about 0, or less than 0, e.g. in the range of about 0 to about 0.4.

In case the biomarker for mitochondrial resilience and/or functionality is ATP, as described herein, the blood sample is, preferably, determined to be abnormal if the level of ATP is less than about 170% higher, less than about 150% higher, less than about 130% higher, less than about 110% higher, less than about 100% higher, less than about 80% higher, less than about 60% higher, less than about 40% higher, less than about 20% higher or less than about 10% higher than the ATP level determined for a blood sample that has been taken from the subject before the physical exercise.

Thus, the blood sample may be also determined to be abnormal if the normalized stress level of ATP, as described herein, is less than about 1.7, less than about 1.5, less than about 1.3, less than about 1.1, less than about 1, less than about 0.8, less than about 0.6, less than about 0.4, less than about 0.2, or less than about 0.1, e.g. in the range of about 0.1 to 1.7.

As regards, in particular, the biomarkers for mitochondrial resilience and/or functionality (e.g. TFAM and/or ATP), measuring the levels of these biomarkers, the sample (esp. the blood sample), the subject (e.g. an athlete or a subject that is undergoing or scheduled for rehabilitation), and the physical exercise, the same applies, as described herein in context of the inventive method of predicting a muscle, tendon and/or ligament injury in a subject, *mutatis mutandis.*

Furthermore, as described herein, the stress level of a biomarker for mitochondrial resilience and/or functionality refers, in particular, to a level of the biomarker measured after a physical exercise of a subject. The level of a biomarker for mitochondrial resilience and/or functionality measured before the physical exercise (e.g. in a blood sample taken from the subject before the physical exercise) refers, in particular, to the "baseline level" of the biomarker, as described herein.

A strong (e.g. ≥20%, ≥40%, ≥60%, ≥80%, ≥100%, ≥150% or ≥200%) increase of the level of a biomarker for mitochondrial resilience and/or functionality (such as TFAM or ATP), as described herein, especially in blood (more specifically in a leucocyte such as a PBMC) after a physical exercise may be associated with a good mitochondrial repair, mitochondrial biogenesis, mitochondrial transcription and/or cellular respiration, and/or a low likelihood that a muscle, tendon and/or ligament injury will occur in the subject, as described herein. A strong (e.g. ≥20%, ≥40%, ≥60%, ≥80%, ≥100%, ≥150% or ≥200%) increase of the level of the biomarker after a physical exercise leads, in particular, to a "normal stress level" of the biomarker.

In contrast, a poor (e.g. <20%, <40%, <60%, <80%, <100%, <150% or <200%) increase of the level of a biomarker for mitochondrial resilience and/or functionality (such as TFAM or ATP), as described herein, especially in blood (more specifically in a leucocyte such as a PBMC) after a physical exercise may be associated with a poor mitochondrial repair, mitochondrial biogenesis, mitochondrial transcription and/or cellular respiration, and/or a high likelihood that a muscle, tendon and/or ligament injury will occur in the subject, as describe herein. A poor (e.g. <20%, <40%, <60%, <80%, <100%, <150% or <200%) increase of the level of a biomarker for mitochondrial resilience and/or functionality (such as TFAM or ATP) leads, in particular, to an "abnormal stress level" of the biomarker, as described herein.

Furthermore, the method of detecting an abnormal stress level of a biomarker for mitochondrial resilience and/or functionality in a blood sample from a subject, as described herein, may further comprises as step (c) reporting to said subject whether said sample is determined to be abnormal or normal.

Moreover, an abnormal stress level of at least one biomarker for mitochondrial resilience and/or functionality in the blood sample may be indicative of a high likelihood that a muscle, tendon and/or ligament injury will occur in the subject, as described herein.

Preferably herein, e.g., in context of the method of detecting an abnormal level of mature and/or active TFAM protein in a sample from a patient, the term about means +/- 20%, preferably +/- 10%, of the given value.

Furthermore, the present invention relates to an analytic method comprising a step of measuring the levels of (i) at least one biomarker for mitochondrial resilience and/or functionality and (i) of at least one cytokine in a blood sample from a subject.

The invention also relates to an analytic method comprising a step of measuring the levels of TFAM and ATP, and optionally of at least one cytokine, in a blood sample from a subject.

As regards, in particular, the biomarkers for mitochondrial resilience and/or functionality (e.g. TFAM and/or ATP), the cytokines, the levels of said biomarkers and cytokines, measuring the levels of these biomarkers and cytokines, the sample (esp. the blood sample), the subject (e.g. an athlete or a subject that is undergoing or scheduled for rehabilitation), the same applies, as described herein in context of the inventive method of predicting a muscle, tendon and/or ligament injury in a subject, *mutatis mutandis,* for the analytic methods of the invention.

The measured levels of at least one biomarker for mitochondrial resilience and/or functionality (e.g., TFAM and/or ATP) and, optionally, of at least one cytokine may be indicative of a certain likelihood that a muscle, tendon and/or ligament injury will occur in the subject, may be used for determining whether a blood sample is abnormal, and/or may be used for predicting a muscle, tendon and/or ligament injury in a subject, as described herein.

Furthermore, the measured levels of at least one biomarker for mitochondrial resilience and/or functionality (e.g., TFAM and/or ATP) and, optionally, of at least one cytokine may be used for preventing a muscle, tendon and/or ligament injury in a subject, for optimizing the performance and/or training of an athlete, and/or for optimizing the rehabilitation of a patient, as described herein.

The present invention also relates to a method of preventing a muscle, tendon and/or ligament injury in a subject, said method comprising
(a) predicting a muscle, tendon and/or ligament injury in a subject, as described herein, and
(b) instructing the subject to perform no or only an easy physical exercise, and/or to discontinue or reduce a physical exercise, as described herein, in case there is a high likelihood that a muscle, tendon and/or ligament injury will occur in the subject, as described herein.

Furthermore, the present invention relates to a method of preventing a muscle, tendon and/or ligament injury in a subject, said method comprising
(a) detecting an abnormal stress level of a biomarker for mitochondrial resilience and/or functionality in a blood sample from a subject, as described herein, and
(b) instructing the subject to perform no or only an easy physical exercise, and/or to discontinue or reduce a physical exercise, as described herein, in case an abnormal stress level of a biomarker for mitochondrial resilience and/or functionality has been detected in a blood sample from the subject, as described herein.

Additionally, the present invention relates to a method of optimizing the performance and/or training of an athlete, said method comprising
(a) predicting a muscle, tendon and/or ligament injury in the athlete, as described herein; and
(b)
   (i) instructing the athlete to perform no or only an easy physical exercise, and/or to discontinue or reduce a physical exercise, as described herein, in case there is a high likelihood that a muscle, tendon and/or ligament injury will occur in the subject, as described herein; and/or
   (ii) instructing the athlete to perform a physical exercise, preferably a strenuous physical exercise, and/or to continue or extend a physical exercise, as described herein, in case there is a low likelihood that a muscle, tendon and/or ligament injury will occur in the subject, as described herein.

Furthermore, the present invention relates to a method of optimizing the performance and/or training of an athlete, said method comprising
(a) an abnormal stress level of a biomarker for mitochondrial resilience and/or functionality in a blood sample from a subject, as described herein; and
(b)
   (i) instructing the athlete to perform no or only an easy physical exercise, and/or to discontinue or reduce a physical exercise, as described herein, in case an abnormal stress level of a biomarker for mitochondrial resilience and/or functionality has been detected in a blood sample from the athlete, as described herein; and/or
   (ii) instructing the athlete to perform a physical exercise, preferably a strenuous physical exercise, and/or to continue or extend a physical exercise, as described herein, in case no abnormal stress level (i.e., a normal stress level) of a biomarker for mitochondrial resilience and/or functionality has been detected in a blood sample from the athlete, as described herein.

The present invention also relates to a method of optimizing the rehabilitation of a patient, said method comprising
(a) predicting a muscle, tendon and/or ligament injury in a patient that is undergoing or scheduled for rehabilitation, as described herein; and
(b)
   (i) instructing the patient to perform no or only an easy physical exercise, and/or to discontinue or reduce a physical exercise, as described herein, in case there is a high likelihood that a muscle, tendon and/or ligament injury will occur in the patient, as described herein; and/or
   (ii) instructing the patient to perform a physical exercise, preferably a strenuous physical exercise, and/or to continue or extend a physical exercise, as described herein, in case there is a low likelihood that a muscle, tendon and/or ligament injury will occur in the patient, as described herein.

The present invention also relates to a method of optimizing the rehabilitation of a patient, said method comprising
(a) detecting an abnormal stress level of a biomarker for mitochondrial resilience and/or functionality in a blood sample from a patient that is undergoing or scheduled for rehabilitation, as described herein; and
(b)
   (i) instructing the patient to perform no or only an easy physical exercise, and/or to discontinue or reduce a physical exercise, as described herein, in case an abnormal stress level of a biomarker for mitochondrial resilience and/or functionality has been detected in a blood sample from the patient, as described herein; and/or
   (ii) instructing the patient to perform a physical exercise, preferably a strenuous physical exercise, and/or to continue or extend a physical exercise, as described herein, in case no abnormal stress level of a biomarker for mitochondrial resilience and/or functionality has been detected in a blood sample from the patient, as described herein.

### Items

Furthermore, the present invention relates to the following items:
1. A method of predicting a muscle, tendon and/or ligament injury in a subject, said method comprising measuring the level of at least one biomarker for mitochondrial resilience and/or functionality in a blood sample from the subject,
   (i) wherein (a) level(s) of said biomarker(s) below (a) respective threshold(s) is/are indicative of a high likelihood that a muscle, tendon and/or ligament injury will occur in the subject, and/or
   (ii) wherein (a) level(s) of said biomarker(s) equal to or above (a) respective threshold(s) is/are indicative of a low likelihood that a muscle, tendon and/or ligament injury will occur in the subject.
2. The method of item 1, wherein the level(s) of said biomarker(s) for mitochondrial resilience and/or functionality is/are measured in at least one leukocyte such as a peripheral blood mononuclear cell (PBMC), of said blood sample.
3. The method of item 1 or 2, wherein said biomarker(s) for mitochondrial resilience and/or functionality comprise(s) mitochondrial transcription factor A (TFAM) and/or adenosine triphosphate (ATP); and, preferably, wherein the TFAM is comprised in a TFAM-TFB2M complex.
4. The method of item 3, wherein the level of TFAM corresponds to
   (I) the number of interactions of TFAM protein with TFB2M protein,
   (II) the amount of mature TFAM protein, and/or
   (III) the ratio of the amount of mature TFAM protein over the amount of immature TFAM protein;
   preferably to the number of interactions of TFAM protein with TFB2M protein.
5. The method of item 4, wherein measuring the interactions of TFAM protein with TFB2M protein, comprises the steps of
   (a) contacting the sample with a pair of antigen-binding molecules, preferably antibodies,
      wherein one of said antigen-binding molecules specifically binds TFAM protein, and wherein the other of said antigen-binding molecules specifically binds TFB2M protein, and
   (b) generating a detectable signal when said two binding molecules are in close proximity to each other.
6. The method of item 4 or 5, wherein the interactions of TFAM protein with TFB2M protein are measured by a proximity ligation assay (PLA) and/or a proximity-dependent initiation of hybridization chain reaction (proxHCR).
7. The method of any one of items 3 to 6, wherein the level of TFAM corresponds to the amount of mature TFAM protein in mitochondria.
8. The method of any one of items to 4 to 7, wherein
   (i) the mature TFAM protein is a human mature TFAM protein with the sequence set forth in SEQ ID NO:4 or SEQ ID NO:8, preferably SEQ ID NO:4, and/or a protein that has at least 60%, 70%, 80% or 90% sequence identity to the sequence set forth in SEQ ID NO:4;
   (ii) the immature TFAM protein is a human immature TFAM protein with the sequence set forth in SEQ ID NO:2 or SEQ ID NO:6, preferably SEQ ID NO:2, and/or a protein that has at least 60%, 70%, 80% or 90% sequence identity to the sequence set forth in SEQ ID NO:2;
   (iii) the immature TFAM protein contains a N-terminal part with the sequence set forth in SEQ ID NO:22 or a sequence that has at least 60%, 70%, 80% or 90% sequence identity to the sequence set forth in SEQ ID NO:22; and/or
   (iv) the mature TFAM protein does not contain at the N-terminus the sequence set forth in SEQ ID NO:22 or a sequence that has at least 60%, 70%, 80% or 90% sequence identity to the sequence set forth in SEQ ID NO:22.
9. The method of any one of items 3 to 8, wherein the level of ATP is measured by an assay, wherein luciferin, in particular D-luciferin, is converted to oxyluciferin in the presence of oxygen and ATP.
10. The method of any one of items 1 to 9, wherein the biomarker(s) for mitochondrial resilience and/or functionality is/are associated positively with mitochondrial repair, mitochondrial biogenesis, mitochondrial transcription and/or cellular respiration.
11. The method of any one of items 1 to 10, wherein said method further comprises measuring the level of at least one cytokine in a blood sample from said subject, preferably in the serum or plasma, more preferably the serum, of said blood sample.
12. The method of item 11, wherein said cytokine(s) comprise(s) at least one interleukin, at least one interferon, and/or TNF-o.
13. The method of item 11 or 12, wherein said cytokine(s) comprise(s) IL-6, IL-8, IL-10 and/or TNF-o.
14. The method of item 13, wherein
   (i) a level of IL-8 below a respective threshold is indicative of a high likelihood that a muscle, tendon and/or ligament injury will occur in the subject, and/or
      wherein
   (ii) a level of IL-8 equal to or above a respective threshold is indicative of a low likelihood that a muscle, tendon and/or ligament injury will occur in the subject.
15. The method of item 13 or 14, wherein
   (i) (a) level(s) of IL-6, IL-10 and/or TNF-o below (a) respective threshold(s) is/are indicative of a low likelihood that a muscle, tendon and/or ligament injury will occur in the subject, and/or
   (ii) wherein (a) level(s) of IL-6, IL-10 and/or TNF-o equal to or above (a) respective threshold(s) is/are indicative of a high likelihood that a muscle, tendon and/or ligament injury will occur in the subject.
16. The method of any one of items 1 to 15, wherein the level(s) of said biomarker(s) for mitochondrial resilience and/or functionality and/or of said cytokine(s) is/are measured before a physical exercise of the subject (baseline level) and/or after a physical exercise of the subject (stress level), preferably at least after the physical exercise, more preferably before and after the physical exercise.
17. The method of any one of items 1 to 16, wherein the level of at least one of said biomarker(s) for mitochondrial resilience and/or functionality corresponds to a normalized level that is calculated by normalizing the stress level(s) of said biomarker(s) to the respective baseline level(s), preferably according to the following formula: (stress level - baseline level) / baseline level.
18. The method of item 16 or 17, wherein TFAM baseline and stress levels are measured, wherein the TFAM level corresponds to a normalized TFAM level, and wherein the normalized TFAM level is calculated by the following formula: (TFAM stress level - TFAM baseline level) / TFAM baseline level).
19. The method of item 18, wherein the threshold of the normalized TFAM level is in the range of about 0 to 0.4, preferably about 0.1 to 0.3, preferably about 0.2
20. The method of any one of items 16 to 19, wherein ATP baseline and stress levels are measured, wherein the ATP level corresponds to a normalized ATP level, and wherein the normalized ATP level is calculated by the following formula: (ATP stress level - ATP baseline level) / ATP baseline level).
21. The method of item 20, wherein the threshold of the normalized ATP level is in the range of about 0.1 to 1.7, preferably about 0.6 to 1.1, preferably about 0.9.
22. The method of any one of items 11 to 21, wherein the level(s) of said cytokine(s), e.g., of TNF-o, is/are measured at least after a physical exercise of the subject (stress level) and wherein the level(s) of said cytokines(s) correspond to the stress level(s) of said cytokine(s).
23. The method of any one of items 11 to 21, wherein the level(s) of said cytokine(s), e.g., of IL-6, IL-8 and/or IL-10, is/are measured before a physical exercise of the subject (baseline level) and after the physical exercise of the subject (stress level), and wherein the level(s) of said cytokines(s) correspond to the maximum level selected from the respective baseline and stress levels.
24. The method of any one of items 14 to 23, wherein the threshold of the IL-8 level is about 30 pg/ml, preferably about 27 pg/ml.
25. The method of any one of items 15 to 24, wherein the threshold of the IL-6 level is about 1 pg/ml, preferably about 1.3 pg/ml; the threshold of the IL-10 level is about 1 pg/ml, preferably about 1.4 pg/ml; and/or the threshold of the TNF-o level is about 4 pg/ml, preferably about 3.7 pg/ml.
26. The method of any one of items 1 to 25, wherein a score is calculated based on
   (a) the levels of at least two of said biomarkers for mitochondrial resilience and/or functionality, preferably TFAM and ATP, and optionally the level(s) of at least one of said cytokine(s), preferably IL-6, IL-8, IL-10 and/or TNF-o; or
   (b) the level(s) of at least one of said biomarkers for mitochondrial resilience and/or functionality, preferably TFAM and/or ATP, and the level(s) of at least one of said cytokine(s), preferably IL-6, IL-8, IL-10 and/or TNF-o;
   and wherein
   (i) a score below a respective threshold is indicative of a low likelihood that a muscle, tendon and/or ligament injury will occur in the subject, and/or
   (ii) a score equal to or above a respective threshold is indicative of a high likelihood that a muscle, tendon and/or ligament injury will occur in the subject.
27. The method of item 26, wherein (a) level(s) of TFAM, ATP and/or IL-8 below the respective threshold(s) and/or (a) level(s) of IL-6, IL-10 and/or TNF-α equal to or higher than the respective threshold(s) positively add(s) to the score.
28. The method of any one of items 16 to 27, wherein the physical exercise comprises an athletic training, and/or a rehabilitation exercise.
29. The method of any one of items 1 to 28, wherein said subject is a human.
30. The method of any one of items 1 to 29, wherein the subject is an athlete such as a football/soccer player, preferably a professional athlete.
31. The method of any one of items 1 to 30, wherein the subject is undergoing or is scheduled for rehabilitation.
32. The method of any one of items 1 to 31, wherein the subject is a patient that had or has a stroke, a heart attack, sepsis, long COVID, or a muscle, tendon and/or ligament injury, preferably a patient that is undergoing or is scheduled for rehabilitation due to a stroke, a heart attack, sepsis, long COVID, or a muscle, tendon and/or ligament injury.
33. The method of any one of items 1 to 32, wherein the injury is a muscle injury, and preferably wherein the muscle comprises at least one skeletal muscle such as *M. Quadriceps Femoris, M. Biceps Femoris, M. Tibialis anterior, M. Fibularis longus, M. Fibularis brevis, M. Soleus* and/or *M. Gastrocnemius.*
34. The method of any one of items 1 to 33, wherein the muscle injury is associated with a reduced muscle strength.
35. The method of any one of items 1 to 34, wherein the likelihood that a muscle, tendon and/or ligament injury will occur in the subject is the likelihood that a muscle, tendon and/or ligament injury will occur in the subject within the next 14, 10 or 7 days, preferably within the next 10 days.
36. The method of any one of items 1 to 35, wherein a high likelihood that a muscle, tendon and/or ligament injury will occur in the subject further indicates that the subject is to perform no or only an easy physical exercise, and/or that the subject is to discontinue or reduce a physical exercise; and/or wherein a low likelihood that a muscle, tendon and/or ligament injury will occur in the subject further indicates that the subject can perform a physical exercise, preferably a strenuous physical exercise, and/or that the subject can continue or extend a physical exercise.
37. The method of item 36, wherein the physical exercise comprises an athletic training, and/or a rehabilitation exercise.
38. The method of item 36 or 37, wherein the subject is an athlete; and
   wherein a high likelihood that a muscle, tendon and/or ligament injury will occur in the subject further indicates that the athlete is to perform no or only an easy athletic training, and/or that the athlete is to discontinue or reduce an athletic training, and/or
   wherein a low likelihood that a muscle, tendon and/or ligament injury will occur in the subject further indicates that the athlete can perform an athletic training, preferably a strenuous athletic training, and/or that the athlete can continue or extend an athletic training.
39. The method of item 36 or 37, wherein the subject is undergoing or is scheduled for rehabilitation; and
   wherein a high likelihood that a muscle, tendon and/or ligament injury will occur in the subject further indicates that the subject is to perform no or only an easy rehabilitation exercise, and/or that the subject is to discontinue or reduce a rehabilitation exercise, and/or
   wherein a low likelihood that a muscle, tendon and/or ligament injury will occur in the subject further indicates that the subject can perform a rehabilitation exercise, preferably a strenuous rehabilitation exercise, and/or that the subject can continue or extend a rehabilitation exercise.
40. The method of any one of items 1 to 39 which is useful to prevent a muscle, tendon and/or ligament injury in the subject.
41. The method of item 40, wherein a muscle, tendon and/or ligament injury is prevented by indicating to the subject to perform no or only an easy physical exercise, and/or to discontinue or reduce a physical exercise when there is a high likelihood that a muscle, tendon and/or ligament injury will occur.
42. A kit comprising at least one means for measuring the level(s) of at least one biomarker for mitochondrial resilience and/or functionality, and optionally of at least one cytokine, in a blood sample, as defined in any one of items 1 to 25.
43. The kit of item 42, comprising an antigen-binding molecule, preferably an antibody, specifically binding TFAM protein, and an antigen-binding molecule, preferably an antibody, specifically binding TFB2M protein.
44. The kit of item 42 or 43, comprising luciferin and/or a luciferase, preferably wherein the luciferin is D-luciferin.
45. The kit of any one of items 42 to 44, comprising an antigen-binding molecule, preferably an antibody, specifically binding IL-6, an antigen-binding molecule, preferably an antibody, specifically binding IL-8, an antigen-binding molecule, preferably an antibody, specifically binding IL-10, and/or an antigen-binding molecule, preferably an antibody, specifically binding TNF-o.
46. The kit of any one of items 42 to 45 for measuring the level of at least one biomarker for mitochondrial resilience and/or functionality, and optionally of at least one cytokine, in a blood sample.
47. The kit of any one of items 42 to 46 for use in a method of any one of items 1 to 41.
48. The kit of any one of items 42 to 47, further comprising a leaflet or brochure with instructions for carrying out the method according to any one of items 1 to 41.
49 Use of the kit of any one of items 42 to 48 for measuring the level of at least one biomarker for mitochondrial resilience and/or functionality, and optionally of at least one cytokine, in a blood sample, as defined in any one of items 1 to 25.
50. Use of the kit of any one of items 42 to 48 in a method of predicting a muscle, tendon and/or ligament injury in a subject according to any one of items 1 to 41.
51. A method of detecting an abnormal stress level of a biomarker for mitochondrial resilience and/or functionality in a blood sample from a subject, wherein said method comprises
   (a) measuring the level of at least one biomarker for mitochondrial resilience and/or functionality in a blood sample from the subject after a physical exercise of the subject (stress level), and
   (b) determining whether the blood sample is abnormal, wherein the blood sample is determined to be abnormal if the level of at least one biomarker for mitochondrial resilience and/or functionality is less than double, less than about 80% higher, less than about 60% higher, less than about 40% higher, less than about 20% higher, similar or lower than the level(s) determined for a blood sample that has been taken from the subject before the physical exercise (baseline level(s)).
52. The method of item 51, wherein the method further comprises as step I reporting to said subject whether said sample is determined to be abnormal or normal.
53. The method of item 51 or 52, wherein an abnormal stress level of at least one biomarker for mitochondrial resilience and/or functionality in the blood sample is indicative of a high likelihood that a muscle, tendon and/or ligament injury will occur in the subject.
54. A method of preventing a muscle, tendon and/or ligament injury in a subject, said method comprising
   (a) predicting a muscle, tendon and/or ligament injury in a subject according to the method of any one of items 1 to 50, or detecting an abnormal stress level of a biomarker for mitochondrial resilience and/or functionality in a blood sample from a subject according to the method of any one of items 51 to 53, and
   (b) instructing the subject to perform no or only an easy physical exercise, and/or to discontinue or reduce a physical exercise in case there is a high likelihood that a muscle, tendon and/or ligament injury will occur in the subject or in case an abnormal stress level of a biomarker for mitochondrial resilience and/or functionality has been detected in a blood sample from the subject.
55. A method of optimizing the performance and/or training of an athlete, said method comprising
   (a) predicting a muscle, tendon and/or ligament injury in the athlete according to the method of any one of items 1 to 50, or detecting an abnormal stress level of a biomarker for mitochondrial resilience and/or functionality in a blood sample from a subject according to the method of any one of items 51 to 53; and
   (b)
      (i) instructing the athlete to perform no or only an easy physical exercise, and/or to discontinue or reduce a physical exercise in case there is a high likelihood that a muscle, tendon and/or ligament injury will occur in the subject or in case an abnormal stress level of a biomarker for mitochondrial resilience and/or functionality has been detected in a blood sample from the athlete; and/or
      (ii) instructing the athlete to perform a physical exercise, preferably a strenuous physical exercise, and/or to continue or extend a physical exercise in case there is a low likelihood that a muscle, tendon and/or ligament injury will occur in the subject or in case no abnormal stress level of a biomarker for mitochondrial resilience and/or functionality has been detected in a blood sample from the athlete.
56. A method of optimizing the rehabilitation of a patient, said method comprising
   (a) predicting a muscle, tendon and/or ligament injury in a patient that is undergoing or scheduled for rehabilitation according to the method of any one of items 1 to 50, or detecting an abnormal stress level of a biomarker for mitochondrial resilience and/or functionality in a blood sample from a patient that is undergoing or scheduled for rehabilitation according to the method of any one of items 51 to 53; and
   (b)
      (i) instructing the patient to perform no or only an easy physical exercise, and/or to discontinue or reduce a physical exercise in case there is a high likelihood that a muscle, tendon and/or ligament injury will occur in the patient or in case an abnormal stress level of a biomarker for mitochondrial resilience and/or functionality has been detected in a blood sample from the patient; and/or
      (ii) instructing the patient to perform a physical exercise, preferably a strenuous physical exercise, and/or to continue or extend a physical exercise in case there is a low likelihood that a muscle, tendon and/or ligament injury will occur in the patient or in case no abnormal stress level of a biomarker for mitochondrial resilience and/or functionality has been detected in a blood sample from the patient.
57. A method of predicting the development of the performance and/or strength of a subject, said method comprising measuring the level of at least one biomarker for mitochondrial resilience and/or functionality in a blood sample from the subject,
   (i) wherein (a) level(s) of said biomarker(s) outside of (a) respective corridor(s) is/are predictive of no or a low increase of the performance and/or strength of the subject, and/or
   (ii) wherein (a) level(s) of said biomarker(s) within (a) respective corridor(s) is/are predictive of a strong increase of the performance and/or strength of the subject.
58. The method of item 57, wherein the subject is performing or has performed a physical exercise, preferably an athletic training.
59. The method of item 57 or 58, wherein the level(s) of said biomarker(s) for mitochondrial resilience and/or functionality is/are measured according to any one of items 2 and 5 to 9, and/or wherein said biomarker(s) for mitochondrial resilience and/or functionality are as defined in any one of items 3, 4 and 10.
60. The method of any one of items 57 to 59, wherein said method further comprises measuring the level of at least one cytokine in a blood sample from said subject according any one of items 11 to 13.
61. The method of item 60, wherein (a) level(s) of said cytokine(s) outside of (a) respective corridor(s) may be predictive of no or a low increase of the performance and/or strength of the subject, and/or (a) level(s) of said cytokine(s) within (a) respective corridor(s) may be predictive of a strong increase of the performance and/or strength of the subject.
62. A method of determining the performance level and/or strength level of a subject, said method comprising measuring the level of at least one biomarker for mitochondrial resilience and/or functionality in a blood sample from the subject,
   (i) wherein (a) level(s) of said biomarker(s) outside of (a) respective corridor(s) is/are indicative of a low performance and/or strength level of the subject, and/or
   (ii) wherein (a) level(s) of said biomarker(s) within (a) respective corridor(s) is/are indicative of a high performance and/or strength level of the subject.
63. The method of item 62, wherein the level(s) of said biomarker(s) for mitochondrial resilience and/or functionality is/are measured according to any one of items 2 and 5 to 9, and/or wherein said biomarker(s) for mitochondrial resilience and/or functionality are as defined in any one items 3, 4 and 10.
64. The method of item 62 or 63, wherein said method further comprises measuring the level of at least one cytokine in a blood sample from said subject according any one of items 11 to 13.
65. The method of item 64, wherein (a) level(s) of said cytokine(s) outside of (a) respective corridor(s) may be indicative of a low performance and/or strength level of the subject, and/or (a) level(s) of said cytokine(s) within (a) respective corridor(s) may be indicative of a high performance and/or strength level of the subject.
66. The method of any one of items 57 to 65, wherein the level(s) of said biomarker(s) for mitochondrial resilience and/or functionality and/or of said cytokine(s) is/are measured before a physical exercise of the subject (baseline level) and/or after a physical exercise of the subject (stress level), preferably at least after the physical exercise, more preferably before and after the physical exercise.
67. The method of any one of items 57 to 66, wherein the level of at least one of said biomarker(s) for mitochondrial resilience and/or functionality corresponds to a normalized level that is calculated by normalizing the stress level(s) of said biomarker(s) to the respective baseline level(s), preferably according to the following formula: (stress level - baseline level) / baseline level.
68. The method of any one of items 57 to 67, wherein TFAM baseline and stress levels are measured, wherein the TFAM level corresponds to a normalized TFAM level, and wherein the normalized TFAM level is calculated by the following formula: (TFAM stress level - TFAM baseline level) / TFAM baseline level); and/or wherein ATP baseline and stress levels are measured, wherein the ATP level corresponds to a normalized ATP level, and wherein the normalized ATP level is calculated by the following formula: (ATP stress level - ATP baseline level) / ATP baseline level).
69. The method of any one of items 66 to 68, wherein the physical exercise comprises an athletic training.
70. The method of any one of items 57 to 69, wherein the subject is an athlete such as a football/soccer player, preferably a professional athlete.
71. Use of the kit of any one of items 42 to 48 in a method according to any one of items 57 to 70.

The invention is also characterized by the following figures, figure legends and the following non-limiting examples.

### Brief Description of Drawings

**Figure 1****:** Scheme for calculating the Sport Score.
**Figure 2****:** Receiver Operator Characteristic (ROC) analysis of the sports score for prediction of injury showed an AUC of 0.95.
**Figure 3****:** ROC analysis of a score for prediction of injury based on two cytokines alone showed an AUC of 0.80.
**Figure 4****:** ROC analysis of a score for prediction of injury based on ATP and TFAM-TFB2M interaction showed an AUC of 0.83.
**Figure 5****:** ROC analysis of a score for prediction of injury based on IL-6, IL-8 plus ATP and TFAM-TFB2M interaction showed an AUC to 0.91.
**Figure 6****:** ROC analysis based on IL-6 for prediction of injury showed an AUC of 0.73 (other cytokines were comparable).
**Figure 7****:** ROC analysis of a score for prediction of injury showed based on IL-6 plus ATP and TFAM-TFB2M interaction showed an AUC of 0.92.

### Examples

Methods and materials are described herein for use in the present disclosure; other, suitable methods and materials known in the art can also be used. The materials, methods, and examples are illustrative only and not intended to be limiting.

### Example 1 - Predicting sports injuries and assessing performance and strength based on biomarkers in the blood

### 1.1 Materials and Methods

### Cohort and Processing of Blood Samples

From 25 athletes in professional football (i.e., soccer), 30 ml of venous blood was collected before and after a training session. The whole blood was then fractionated into serum, plasma, and immune cells (PBMCs = peripheral blood mononuclear cells). PBMCs and plasma fractions were isolated using Ficoll density gradient centrifugation (GE Healthcare Europe, Freiburg, Germany). The phase containing the PBMCs was collected and washed with PBS. After erythrocyte lysis and various washing steps with PBS, the cells were counted, placed on slides with a Cytospin (Cellspin II, Tharmac, Wiesbaden, Germany), and then fixed with 4% formaldehyde solution. Unused PBMCs were treated with Bambanker and stored at -80°C. The plasma fractions were also stored at -80°C. Serum was obtained using S-Monovettes (Sarstedt, Nümbrecht, Germany) and centrifuged at 4000 g for 4 minutes at room temperature. Leukocytes can be isolated through simple centrifugation from peripheral blood (e.g., finger or earlobe) and used in context of the present invention instead of PBMCs.

### Proximity Ligation Assay (PLA) - TFAM-TFB2M Interaction

To analyze the protein-protein interaction of mitochondrial transcription factor A (TFAM) and mitochondrial transcription factor 2B (TFB2M) in PBMCs, a Proximity Ligation Assay (PLA) was performed. After an initial incubation with primary antibodies against TFAM (1:50, sc-376672, Santa Cruz Biotechnology, Heidelberg, Germany) and TFB2M (1:100, 46-481, ProSci Incorporated, Poway, CA, USA) overnight at 4°C, a second incubation with proximity probes (anti-Mouse Plus; DUO92001 and anti-Goat Minus; DUO92006, Sigma-Aldrich, each 1:5) was carried out for 1 hour at 37°C. Additionally, S3 Splint and S3 Backbone oligonucleotides (Biomers.net; Ulm, Germany) were hybridized and ligated (T4 Ligase, 15224025, Invitrogen, Waltham, MA, USA). After rolling-circle amplification (phi29 DNA Polymerase, EP0094, Thermo Fisher Scientific, Waltham, MA, USA), the products were compacted with an S3-Compaction Oligonucleotide and labeled with a fluorophore for visualization. PBMCs were imaged with a Zeiss Apotome microscope (Zeiss, Oberkochen, Germany). Images were analyzed using ImageJ and CellProfiler to quantify the average PLA signal per cell. PLA signals per cell are a measure of mitochondrial vitality. Instead of PBMCs, also leukocytes in general can be used.

The TFAM-TFB2M interaction plays an important role for mitochondrial resilience and/or functionality, in particular in mitochondrial regeneration, and is thus directly linked to cellular energy supply. To evaluate mitochondrial regeneration, a delta was measured from PLA signals before training (baseline) and after training (stress state) using the formula: [Signals (stress) - Signals (baseline)] / Signals (baseline).

### Measurement of ATP in PBMCs

ATP determination in PBMCs was performed using the CellTiter-Glo^{®} 2.0 Assay (Promega, Walldorf, Germany). This assay is based on the luciferase reaction, which converts D-luciferin to oxyluciferin under oxygen conditions and in the presence of ATP, releasing detectable light. PBMCs were adjusted to 2.5*10^6 cells with PBS buffer (total volume 100 µl). After adding 100 µl CellTiter-Glo^{®}, the samples were incubated for 10 minutes at room temperature in the dark. Luminescence was then measured at 545 nm using a microplate reader (CLARIOstarPLUS, BMG LABTECH, Germany). Data analysis was performed using CLARIOstar Data Analysis Software (BMG LABTECH, Germany). A delta value was calculated from the data as follows: [ATP (stress) / ATP (baseline)] / ATP (baseline). Instead of PBMCs, also leukocytes in general can be used.

### Cytokine Measurement in Blood Serum

The concentration of selected inflammatory and anti-inflammatory cytokines in blood serum was determined using the Luminex^{®} Multiplex Assay (Luminex Corporation, USA). Serum samples were diluted and placed on a Luminex plate. The samples were measured using a Luminex 200 system (Thermo Fisher Scientific, Freiburg, Germany). The following cytokine concentrations were included in the decision tree of the algorithm/score:
- Interleukin 6 (IL-6) (maximum value before and after training)
- Interleukin 8 (IL-8) (maximum value before and after training)
- Interleukin 10 (IL-10) (maximum value before and after training)
- TNF-alpha (TNF-o) (value after training).

### Measurement of Reactive Oxygen Species in Blood Plasma

To measure reactive oxygen species (ROS) in blood plasma, the indicator dye CM-H2DCFDA (Thermo Fisher Scientific/Invitrogen, Freiburg, Germany) was used. CM-H2DCFDA is a chloromethyl derivative whose acetate group is cleaved by esterases and whose chloromethyl group reacts with glutathione and other thiols. Subsequent oxidation leads to fluorescent adducts, which can be measured with a microplate reader. Plasma samples were first diluted 1:100 in PBS buffer. After adding CM-H2DCFDA (final concentration 5 µM), the samples were incubated for 45 minutes at 37°C in the dark. Fluorescence was then measured at 495 nm using a microplate reader (CLARIOstarPLUS, BMG LABTECH, Germany). Data analysis was performed using CLARIOstar Data Analysis Software (BMG LABTECH, Germany).

### Strength, Performance, and Injury Data

Strength (in particular "muscle strength"), performance, and injury data for the data analysis were provided by the football club of the participating athletes. In particular, performance data from training sessions and competitions were retrieved from the Catapult database, and strength data from the athletics coach and injury data from the team doctor were provided, adhering to data protection regulations. Only injuries of a player that occurred without external factors (e.g., opponent hurting the player) were considered. The most common types of injury that happen this way are muscle, tendon and/or ligament injuries. Accordingly, the injuries considered in the analyses were mostly muscle, tendon and/or ligament injuries. Performance data include, in particular, the following:
- Distance and Speed Metrics (e.g., total distance, average velocity, maximal velocity)
- Load and Exertion Metrics (e.g., total player load, heart rate exertion)
- Acceleration and Deceleration Metrics (e.g., maximum effort acceleration, maximum effort deceleration)
- Metabolic Power Metrics (e.g., total effort in metabolic power bands)
- Jump and Impact Metrics (e.g., jump count per minute, total jumps)

Strength data relate include, for example, grip strength, muscle circumference, and/or weight lifted.

### 1.2 Injury data analysis and results

### Creation of a Decision Tree and Development of a "Sport Score" Based on Molecular Biology Data

The data obtained from the inventors' measurements were used to create an algorithm/"Sport Score" to assess whether the injury probability within the next 10 days can be predicted. The "Sport Score" was generated as follows: First, a threshold value was established for each laboratory parameter (ATP, IL-6, IL-8, IL-10, TNF-o, TFAM-TFB2M-PLA) using a receiver operating characteristic analysis (ROC) using the Youden index, which shows the point of best discrimination between injury and non-injury (Table 1).

**Table 1: Parameters and Threshold Values for the Sport Score**

| | Parameter | Threshold | Unit |
|---|---|---|---|
| Cytokine-Score | IL-6 | 1.34 | pg/ml |
| | IL-8 | 27 | pg/ml |
| | IL-10 | 1.43 | pg/ml |
| | TNF-α | 3.66 | pg/ml |
| ATP-Score | ATP | 0.91 | Δ delta |
| PLA-Score | TFAM-TFB2M interaction | 0.19 | Δ delta |

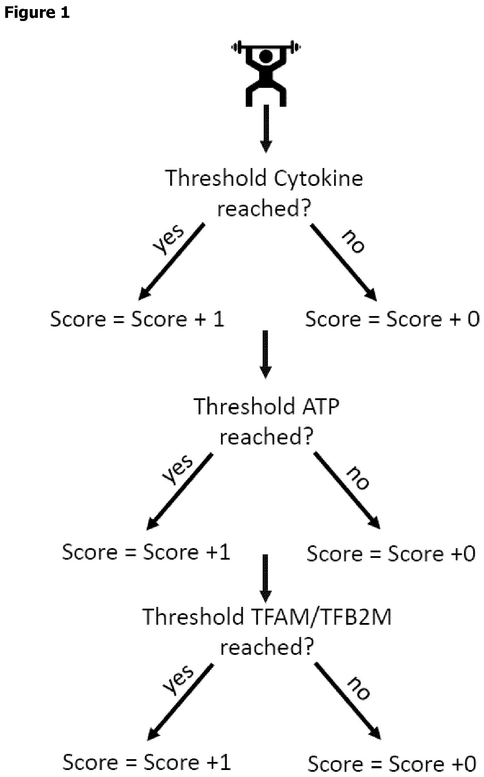

Using the obtained threshold values, a decision tree and a score for each parameter were created (see Fig. 1):
**For IL-6, IL-10, and TNF-α:** If the threshold is exceeded, the score +1 is given. If a parameter remains below the threshold, the score 0 is assigned. **For the IL-8, TFAM-TFB2M, and ATP:** If the threshold is not met, the score +1 is given. If a parameter remains above the threshold, the score 0 is assigned. To determine the "Sport Score," the individual scores are summed up.

### The predictive power of the Sport Score

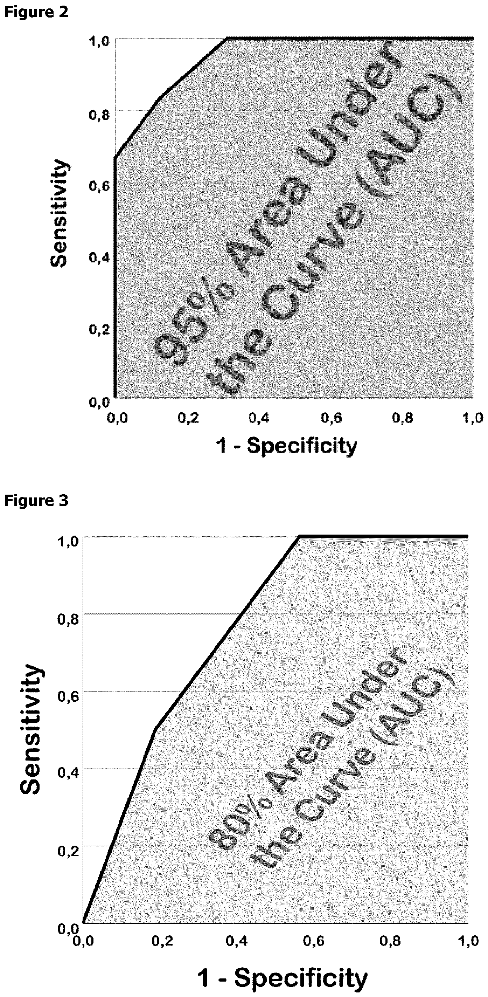

The generated "Sport Score" was then tested for its predictive power in the above-described cohort. A Receiver Operating Characteristic (ROC) analysis was performed and compared with injury data. Remarkably, the AUC of the Sport Score was 95% (Fig. 2). ROC analysis of the Sport Score further revealed that if the Sport Score has a value of 0-1, there is a very low risk of injury in the next 10 days, if it has a value of 2-3, there is a low risk of injury, and if it has a value of 4-6, there is a high risk of injury (Table 2).

**Table 2: Risk assessment based on the "Sport-Score"**

| Sport-Score | 0 | 1 | 2 | 3 | 4 | 5 | 6 |
|---|---|---|---|---|---|---|---|
| Risk for an injury (within the next 10 days) | Very low | Very low | Low | Low | High | High | High |

The inventors further found that the best point discrimination of the Sport Score was the value 4.

Unexpectedly, although only very few injury cases were observed in the short period the study was performed (6 months), a score below 4 could exclude an injury with 93% accuracy, and a score greater than or equal to 4 predicted an injury with 71% accuracy (see Table 3).

**Table 3: Test of the predictive power of the "Sport Score" employing the collected injury data**

| | Injured: no | Injured: yes | Total |
|---|---|---|---|
| Score < 4 (low risk) | **13 (93 %)** | 1 (7 %) | 14 |
| Score >/= 4 (high risk) | 2 (29 %) | **5 (71 %)** | 7 |

### Influence of Mitochondrial Factors (i.e. Biomarkers for Mitochondrial Resilience and/or Functionality) on the Sport Score

In addition to the "Sport Score" which considers all four relevant cytokines as well as TFAM-TFB2M and ATP the inventors generated smaller scores using only some of the parameters of the Sport Score to assess whether these smaller scores still allow to predict injuries.

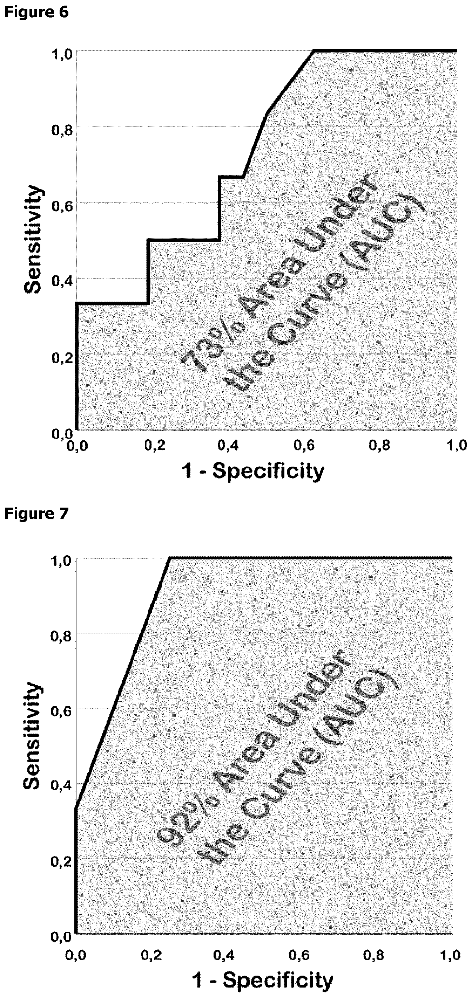

So far, mainly immunological markers (in particular cytokines) have been used to predict injuries in sports. A score based on one of the measured cytokines (IL-6, IL-8, IL-10, TNF-o) alone achieved merely an AUC of up to 73% (Fig. 6), while two of the measured cytokines in combination could achieve an AUC of up to 80% (Fig. 3). Including all four cytokines did not significantly improve the score. It is thus concluded that combining several cytokines in the score provides, surprisingly, only very little additional value.

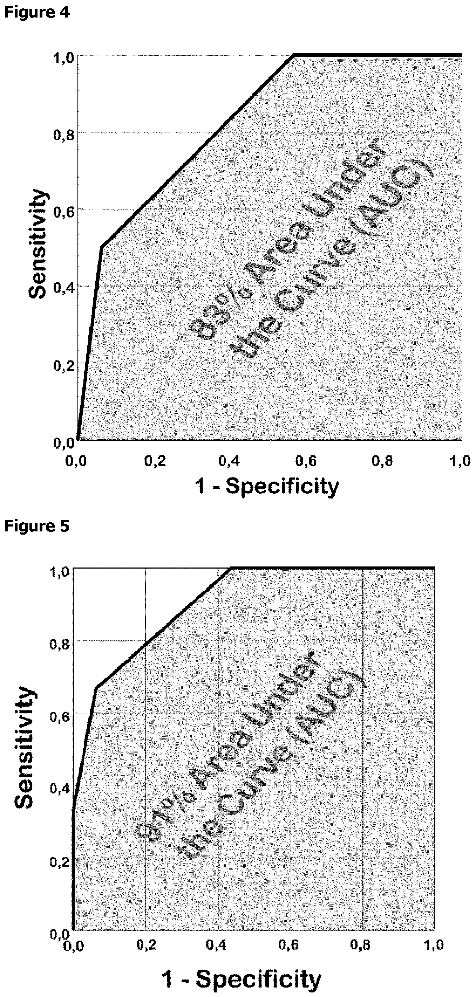

Completely unexpectedly, a score based on the levels of ATP and the TFAM-TFB2M interaction (i.e. two biomarkers for mitochondrial resilience and/or functionality) in leukocytes alone achieved a higher AUC of 83% which was thus improved over a purely cytokine-based score (Fig. 4). While the TFAM-TFB2M interaction (PLA) alone achieved an AUC of 60%, and ATP alone achieved an AUC of 68%, the AUC achieved by the combination of both was significantly higher (83%). Thus, combining the TFAM-TFB2M interaction and ATP provides additional informational value in predicting sports injuries such as muscle injuries.

Further surprisingly, the predictive power of a cytokine (e.g., IL-6, IL-8, IL-10 and/or TNF-o)-based score can be significantly increased by including a biomarker for mitochondrial resilience and/or functionality, in particular ATP. Specifically, a combination of one of the measured cytokines (IL-6, IL-8, IL-10 or TNF-o) with ATP achieved an AUC of 84-92%, i.e., up to 92% which is nearly as high as for the total Sport Score (95%).

Hence, the combination of a cytokine (e.g., a pro-inflammatory or an anti-inflammatory cytokine - it seems little important which one) with a biomarker for mitochondrial resilience and/or functionality such as ATP provides additional informational value in predicting sports injuries such as muscle injuries.

To further increase robustness of the score, it may be advantageous to combine the TFAM-TFB2M interaction, ATP and at least one cytokine for predicting sports injuries. Such scores achieved an AUC of 84-95%, e.g., 92% for IL-6 in combination with ATP and TFAM-TFB2M interaction (Fig. 7), and 95% for the Sport Score (Fig. 2).

### 1.3 Analysis of performance and strength data

Performance and strength metrics will be systematically correlated with the molecular biology parameters, in particular, TFAM-TFB2M interaction, cytokines, ATP, and reactive oxygen species (ROS), as described in Example 1.1.

Endpoints for these metrics will be defined (e.g., an X% increase in average velocity within 4 weeks), and Receiver Operating Characteristic (ROC) analyses, similar to those used for injury prediction, will be employed to evaluate the influence of different molecular variables on these endpoints. This will enable the development of a scoring system that predicts performance improvement, as described in Example 1.2.

Specific ranges (i.e., corridors) within these metrics that reflect an optimal training effect will be identified, leading to measurable performance gains over time. The outer boundaries of these corridors (e.g. the lower boundary for TFAM, ATP or IL-8, or the upper boundary for IL-6, IL-10 and/or TNF-o) are expected to coincide with the thresholds used in injury risk prediction, while the inner boundaries (e.g., the upper boundary for TFAM, ATP or IL-8, or the lower boundary for IL-6, IL-10 and/or TNF-o) will likely indicate thresholds for insufficient training stimuli. Values outside of the corridors may thus predict minimal or no performance improvement, either due to insufficient training or a high propensity for injury.

In addition to evaluating long-term performance trends, the inventors expect some of these molecular parameters to be directly linked to the acute training state of the athletes, reflecting immediate physiological responses rather than long-term adaptations.

## Claims

1. A method of predicting a muscle, tendon and/or ligament injury in a subject, said method comprising measuring the level of at least one biomarker for mitochondrial resilience and/or functionality in a blood sample from the subject,
(i) wherein (a) level(s) of said biomarker(s) below (a) respective threshold(s) is/are indicative of a high likelihood that a muscle, tendon and/or ligament injury will occur in the subject, and/or
(ii) wherein (a) level(s) of said biomarker(s) equal to or above (a) respective threshold(s) is/are indicative of a low likelihood that a muscle, tendon and/or ligament injury will occur in the subject.

2. The method of claim 1, wherein the level(s) of said biomarker(s) for mitochondrial resilience and/or functionality is/are measured in at least one leukocyte such as a peripheral blood mononuclear cell (PBMC), of said blood sample.

3. The method of claim 1 or 2, wherein said biomarker(s) for mitochondrial resilience and/or functionality comprise(s) mitochondrial transcription factor A (TFAM) and/or adenosine triphosphate (ATP); and, preferably, wherein the level of TFAM corresponds to the number of interactions of TFAM protein with TFB2M protein.

4. The method of claim 3, wherein the interactions of TFAM protein with TFB2M protein are measured by a proximity ligation assay (PLA) and/or a proximity-dependent initiation of hybridization chain reaction (proxHCR), preferably a PLA.

5. The method of any one of claims 1 to 4, wherein said method further comprises measuring the level of at least one cytokine in a blood sample from said subject, preferably in the serum of said blood sample; and preferably, wherein said cytokine(s) comprise(s) IL-6, IL-8, IL-10 and/or TNF-α.

6. The method of claim 5, wherein
(i) a level of IL-8 below a respective threshold is indicative of a high likelihood that a muscle, tendon and/or ligament injury will occur in the subject, and/or a level of IL-8 equal to or above a respective threshold is indicative of a low likelihood that a muscle, tendon and/or ligament injury will occur in the subject, and/or
(ii) (a) level(s) of IL-6, IL-10 and/or TNF-o below (a) respective threshold(s) is/are indicative of a low likelihood that a muscle, tendon and/or ligament injury will occur in the subject, and/or (a) level(s) of IL-6, IL-10 and/or TNF-o equal to or above (a) respective threshold(s) is/are indicative of a high likelihood that a muscle, tendon and/or ligament injury will occur in the subject.

7. The method of any one of claims 1 to 6, wherein the level(s) of said biomarker(s) for mitochondrial resilience and/or functionality is/are measured before a physical exercise of the subject (baseline level) and/or after a physical exercise of the subject (stress level), preferably before and after the physical exercise; and/or wherein the level of at least one of said biomarker(s) for mitochondrial resilience and/or functionality corresponds to a normalized level that is calculated by normalizing the stress level(s) of said biomarker(s) to the respective baseline level(s).

8. The method of any one of claims 1 to 7, wherein a score is calculated based on
(a) the levels of at least two of said biomarkers for mitochondrial resilience and/or functionality, preferably TFAM and ATP, and optionally the level(s) of at least one of said cytokine(s), preferably IL-6, IL-8, IL-10 and/or TNF-o; or (b) the level(s) of at least one of said biomarkers for mitochondrial resilience and/or functionality, preferably TFAM and/or ATP, and the level(s) of at least one of said cytokine(s), preferably IL-6, IL-8, IL-10 and/or TNF-o;
and wherein
(i) a score below a respective threshold is indicative of a low likelihood that a muscle, tendon and/or ligament injury will occur in the subject, and/or
(ii) a score equal to or above a respective threshold is indicative of a high likelihood that a muscle, tendon and/or ligament injury will occur in the subject; and, preferably,
wherein (a) level(s) of TFAM, ATP and/or IL-8 below the respective threshold(s) and/or (a) level(s) of IL-6, IL-10 and/or TNF-o equal to or higher than the respective threshold(s) positively add(s) to the score.

9. The method of any one of claims 1 to 8, wherein (i) the subject is an athlete, and optionally, the physical exercise comprises an athletic training, and/or (ii) wherein the subject is undergoing or is scheduled for rehabilitation, and optionally, the physical exercise comprises a rehabilitation exercise.

10. The method of any one of claims 1 to 9, wherein the injury is a muscle injury, preferably of a skeletal muscle.

11. The method of any one of claims 1 to 10, wherein the likelihood that a muscle, tendon and/or ligament injury will occur in the subject is the likelihood that a muscle, tendon and/or ligament injury will occur in the subject within the next 14, 10 or 7 days, preferably within the next 10 days.

12. The method of any one of claims 1 to 11, wherein a high likelihood that a muscle, tendon and/or ligament injury will occur in the subject further indicates that the subject is to perform no or only an easy physical exercise, and/or that the subject is to discontinue or reduce a physical exercise; and/or wherein a low likelihood that a muscle, tendon and/or ligament injury will occur in the subject further indicates that the subject can perform a physical exercise, preferably a strenuous physical exercise, and/or that the subject can continue or extend a physical exercise.

13. A method of predicting the development of the performance and/or strength of a subject, said method comprising measuring the level of at least one biomarker for mitochondrial resilience and/or functionality as defined in claim 1 or 3 in a blood sample from the subject according to any one claims 1, 2, 4 and 7, (i) wherein (a) level(s) of said biomarker(s) outside of (a) respective corridor(s) is/are predictive of no or a low increase of the performance and/or strength of the subject, and/or (ii) wherein (a) level(s) of said biomarker(s) within (a) respective corridor(s) is/are predictive of a strong increase of the performance and/or strength of the subject.

14. A method of determining the performance level and/or strength level of a subject, said method comprising measuring the level of at least one biomarker for mitochondrial resilience and/or functionality as defined in claim 1 or 3 in a blood sample from the subject according to any one claims 1, 2, 4 and 7,
(i) wherein (a) level(s) of said biomarker(s) outside of (a) respective corridor(s) is/are indicative of a low performance and/or strength level of the subject, and/or
(ii) wherein (a) level(s) of said biomarker(s) within (a) respective corridor(s) is/are indicative of a high performance and/or strength level of the subject.

15. A kit comprising (i) an antibody binding TFAM and a means for measuring the level of ATP, (ii) an antibody binding TFAM and an antibody binding a cytokine and/or (iii) a means for measuring the level of ATP, and an antibody binding a cytokine.
